# EUROPEAN PATENT APPLICATION

(11) **EP 4 130 016 A1**
(43) Date of publication of application: **08.02.2023**
(21) Application number: 21776082.6
(22) Date of filing: 26.03.2021
(51) Int. Cl.: C07H 15/08, C07K 16/00

(54) **SUGAR COMPOUND HAVING POLYETHYLENE GLYCOL CHAIN, AND PRECURSOR OF ANTIBODY-DRUG COMPLEX**

(30) Priority: 26.03.2020 JP 2020055760
(71) Applicant: The Noguchi Institute, Tokyo 1730003 (JP)
(72) Inventor: GOTO Kohtaro, Tokyo 173-0003 (JP); MIZUNO Mamoru, Tokyo 173-0003 (JP); MATSUDA Akio, Tokyo 173-0003 (JP); TSUKIMURA Wataru, Tokyo 173-0003 (JP)
(74) Representative: Gille Hrabal Partnerschaftsgesellschaft mbB Patentanwälte
(86) International application number: PCT/JP2021/012997
(87) International publication number: WO 2021/193951

(57) **Abstract**

The object of the present invention is to provide a synthetic method for ADC synthesis with controllable regioselectivity and number of drugs introduced, as well as synthetic intermediates and synthetic raw materials for such methods.

The object can be solved by a sugar compound having a polyethylene glycol chain, of the following general formula (1). 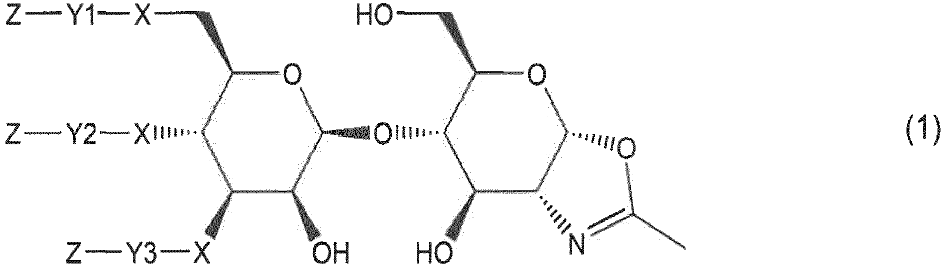 wherein each X is independently a single bond, an oxygen atom, -NH-, -COHN-, -COO-, or a group of formula (5) or (6), (wherein R¹ is trivalent branched hydrocarbon group having 1 to 6 carbon atoms, R² and R³ are each independently an alkylene group having 1 to 3 carbon atoms), any one or more of Y1 to Y3 are present and are independently a PEG chain, a substituted PEG chain, or a PEG chain containing an oxygen atom, -NH-, - COHN-, or -COO- in the main chain, a structure of the PEG chain is linear or branched, and the number of branches is 2 to 10 in the case of branched structure, Z is independently a hydroxy group, a methoxy group, an azido group, a tetrazine group which may be optionally substituted, a norbornene group, a trans-cyclooctene group, a dibenzylcyclooctyl group, or a bicyclo[6.1.0]nona-4-yn-9-ylmethyl group, or a cyanobenzothiazole group which may be optionally substituted, wherein at least one of Z is not a hydroxy group or methoxy group, when any of Y1 to Y3 is not present, Z is hydrogen and X is oxygen, when X is the group of formula (5) or (6), Y1 to Y3 are bonded to each of R² and R³ of the branched chains thereof, when the PEG chain is branched structure, Z is bonded to each of branched chains.

## Description

### TECHNICAL FIELD

The present invention relates to a modified immunoglobulin antibody which is a precursor of antibody-drug conjugates (ADCs), and a sugar compound having a polyethylene glycol chain modified with functional groups (sugar oxazoline compound) which is raw materials for their synthesis.

### BACKGROUND ART

ADCs, wherein drug(s) having cytotoxic activity is/are coupled to antibody drugs that specifically bind to target antigens expressed on cancer cells, have been attracting attention in recent years as next-generation antibody drugs.

For example, however, the method of ADC synthesis in which a drug is attached to the amino group or thiol group of the side chain of an IgG antibody via a linker, is generally used. Thus, since the drug is introduced randomly into the amino group or thiol group at multiple locations on the antibody, a diverse ADC isomers group with a wide distribution in the position and number of drug introductions is obtained. Such ADC isomers group may cause clinical problems such as quality control and drug efficacy (circulating half-life, decreased efficacy, or the like).

As a method to solve this problem, regioselective introduction of drugs using N-linked sugar chains in the Fc region of IgG antibodies as scaffolds has been reported (Non-Patent literatures 1 and 2). These methods can selectively introduce drugs only into the glycan moiety of the antibody. Therefore, these methods have the advantages of higher regioselectivity and controllability of the number of drugs introduced than the conventional method of introducing drugs randomly.

In all of these methods, it is necessary to introduce a functional group into N-acetylneuraminic acid at the non-reducing end of the sugar chain for drug delivery. However, it has been pointed out that N-acetylneuraminic acid may be released from the sugar chains in the blood, and thus there is a risk that the drug is released from the ADC outside the target cells. In addition, the sugar chains used in these methods are prepared from chicken eggs, and therefore allergens derived from eggs are problematic from the viewpoint of GMP in drug manufacturing.

### CITATION LIST

### NON-PATENT LITERATURE

[NON-PATENT LITERATURE 1] F. Tang et al., Org. Biomol. Chem., 14, 9501 (2016)
[NON-PATENT LITERATURE 2] M. Iwamoto et al., Bioconjugate Chem., 29, 2829 (2018)
[NON-PATENT LITERATURE 3] K. Goto et al., Tetrahedron Lett., 61, 151475 (2020)
[NON-PATENT LITERATURE 4] M. Aoyama et al., Mabs, 11,826-836 (2019)

### SUMMARY OF INVENTION

### TECHNICAL PROBLEM

The object of the present invention is to provide a synthetic method for ADC synthesis with controllable regioselectivity and number of drugs introduced, as well as synthetic intermediates and synthetic raw materials for such methods

### SOLUTION TO PROBLEM

As a result of our intensive study to solve the above problem, we have succeeded in developing a sugar oxazoline derivative as a tool for ADC synthesis that has regioselectivity to antibodies and can control the number of drugs to be introduced. Since the sugar oxazoline derivatives of the present invention are fully chemically synthesized, they are suitable for mass synthesis and can overcome the allergenic problem.
Furthermore, the absence of N-acetylneuraminic acid in the molecule is expected to solve the problem of stability in blood.
Moreover, the sugar oxazoline derivative of the present invention can be regioselectively introduced into the sugar moiety of antibodies by using endo-β-N-acetylglucosaminidase (ENGase), which is one of the sugar hydrolyzing enzymes. Thus, regioselectively modified antibodies for drug delivery are synthesized. The resulting modified antibodies could be used as ADC precursors to control the regioselective introduction of drugs into antibodies and the number of drug introductions. Based on the above findings, we have completed this invention.

Accordingly, the present invention relates to:
[1] a sugar compound having a polyethylene glycol chain, of the following general formula (1). 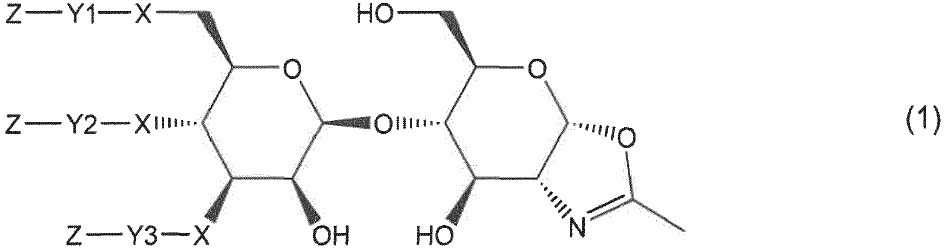 wherein each X is independently a single bond, an oxygen atom, -NH-, -COHN-, -COO-, or a group of formula (5) or (6), 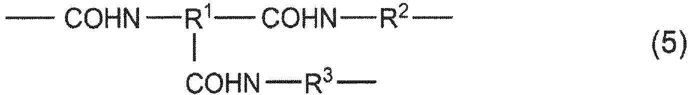 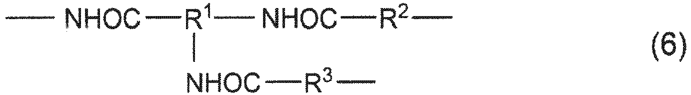 (wherein R' is trivalent branched hydrocarbon group having 1 to 6 carbon atoms, R² and R³ are each independently an alkylene group having 1 to 3 carbon atoms)
   any one or more of Y1 to Y3 are present and are independently a PEG chain, a substituted PEG chain, or a PEG chain containing an oxygen atom, -NH-, -COHN-, or -COO- in the main chain, a structure of the PEG chain is linear or branched, and the number of branches is 2 to 10 in the case of branched structure,
   Z is independently a hydroxy group, a methoxy group, an azido group, a tetrazine group which may be optionally substituted, a norbornene group, a trans-cyclooctene group, a dibenzocyclooctyne group, or a bicyclo[6.1.0]non-4-yn-9-ylmethyl group, or a cyanobenzothiazole group which may be optionally substituted, wherein at least one of Z is not a hydroxy group or methoxy group,
   when any of Y1 to Y3 is not present, Z is hydrogen and X is oxygen,
   when X is the group of formula (5) or (6), Y1 to Y3 are bonded to each of R² and R³ of the branched chains thereof,
   when the PEG chain is branched structure, Z is bonded to each of branched chains,
[2] the sugar compound having a polyethylene glycol chain of item [1], of the following general formula (2). 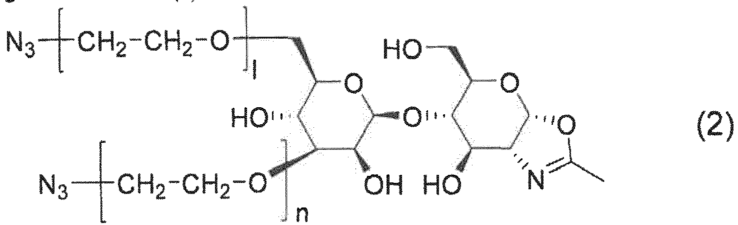 wherein l and n are each independently integers of 2 to 10,
[3] the sugar compound having a polyethylene glycol chain of item [1], selected from the group consisting of a compound 12, a compound represented by the following formula 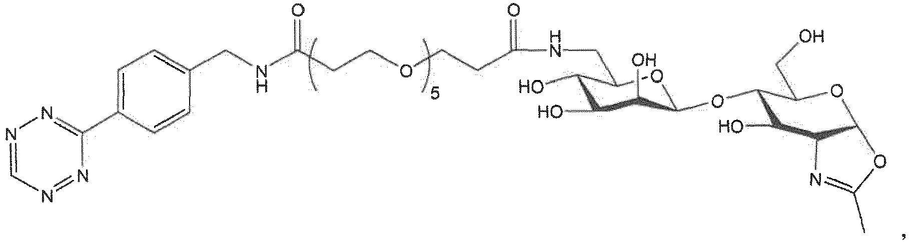 a compound 66, a compound 72, a compound 78, a compound 83, and a compound 94,
[4] a modified immunoglobulin antibody with a sugar compound group having a polyethylene glycol chain represented by the following formula (7), which is derived from the sugar compound of the items [1] to [3], in a sugar-binding site of a side chain of an asparagine residue in a Fc region. 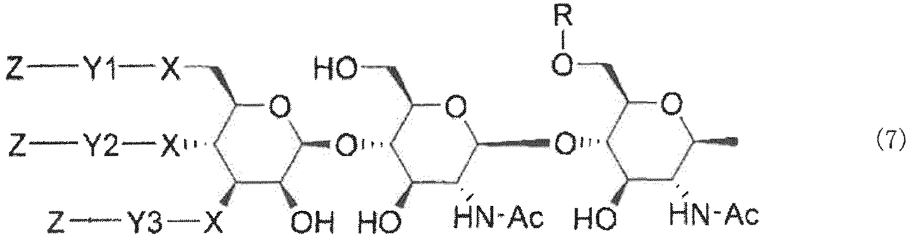 wherein X, Y1 to Y3, and Z are as above mentioned, and R is L-fucose or a hydrogen atom,
[5] the modified immunoglobulin antibody of item [4], represented by the following formula (3), wherein the immunoglobulin antibody is an immunoglobulin G antibody, and the asparagine residue in the Fc region is the 297th asparagine residue in the Fc region. 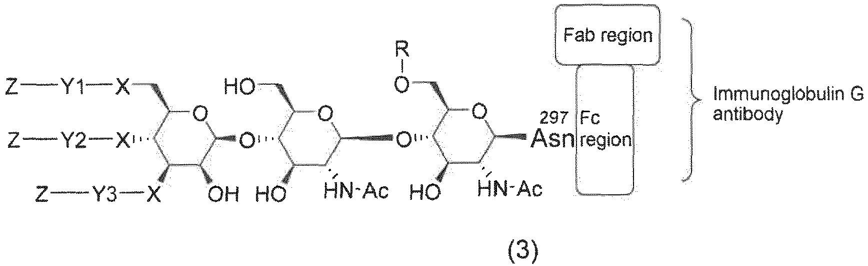 wherein X, Y1 to Y3, and Z are as above mentioned, and R is L-fucose or a hydrogen atom,
[6] the modified immunoglobulin antibody of item [5], represented by the following formula (4), wherein the sugar compound having a polyethylene glycol chain is the sugar compound according to claim 2. 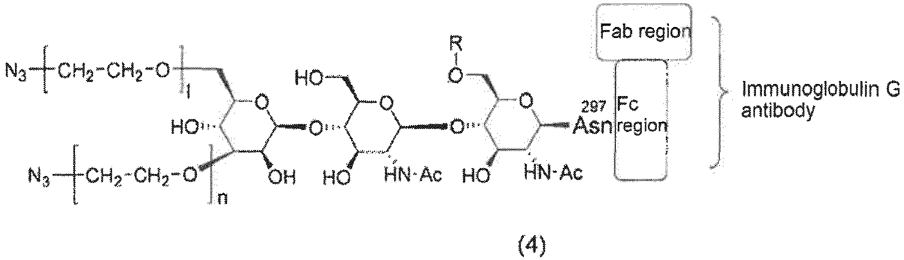 wherein R is L-fucose or a hydrogen atom, and l and n are integers of 2 to 10.

The present specification discloses a sugar oxazoline derivative, and a modified IgG antibody derived therefrom which is ADC precursor, as described below.
<1> A sugar oxazoline derivative connected with a polyethylene glycol (PEG) having a functional group, represented by the following general formula (1). 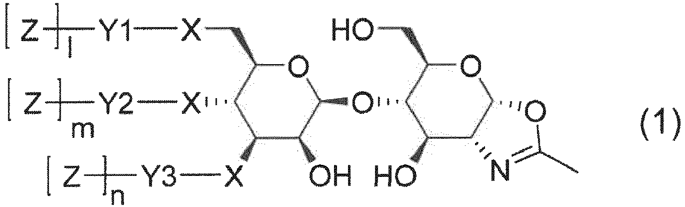
   wherein X is oxygen or an amide group,
   one or more of Y1 to Y3 exist, and Y1 to Y3 is a PEG chain, or a PEG derivative which is derivatized with a functional group and/or an organic group, the structure of the PEG chain and PEG derivative is linear or branched, the number of branches is 2 to 10 in the case of branched structure,
   Z is an azido group or a tetrazine derivative, Z is hydrogen and X is oxygen, in a site where any of Y1 to Y3 is not present,
   When Y1 to Y3 is a linear structure, the number of l, m, n is 1, when Y1 to Y3 is a branched structure, the number l, m, n is equal to or less than the number of branches, the number of l, m, and n is 1 in the site where any of Y1 to Y3 is not present,
   when Y1 to Y3 is a branched structure and Z is two or more in Y1 to Y3, Z may be identical or may not be identical,
   the bonds between X-Y and Y-Z are covalent, and X, Y, and Z need not be identical at each of the positions.
<2> A modified IgG which is ADC precursor, obtained by reacting IgG with a sugar oxazoline derivative represented by general formula (1) in the presence of ENGase, represented by the following formula (3). 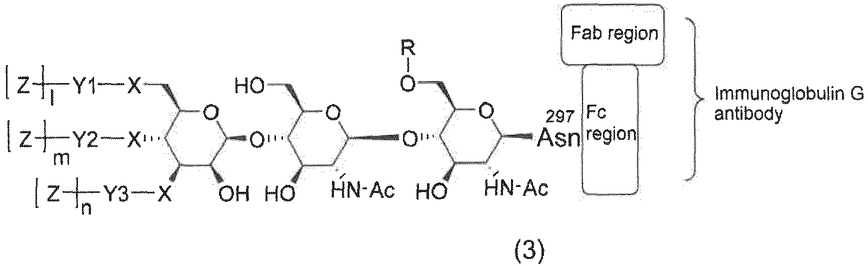 wherein X, Y1-3, Z, l, m, and n are the same as in formula (1), R is L-fucose or a hydrogen atom.

### ADVANTAGEOUS EFFECTS OF INVENTION

According to the present invention, a new modified antibody of ADC precursor can be prepared, which can introduce a fixed number of drugs at a specific position on the antibody, has a chemically and biochemically stable structure, and overcomes the allergenic problem. In addition, by using the sugar compounds of the present invention for the binding of the antibody to the drug, the drug can be introduced into the antibody-drug conjugate under mild conditions (pH, temperature, and the like). Therefore, the antibodies are not degraded during the reaction.

### BRIEF DESCRIPTION OF THE DRAWINGS

FIG. 1 is a photograph of SDS-PAGE showing a solution of the raw material rituximab acceptor (13) and a solution after the transfer reaction with compound 12.
FIG. 2 is an illustration showing the transfer reaction of compound 12 to the rituximab acceptor (13) of rituximab.
FIG. 3 is a photograph of SDS-PAGE showing the introduction of fluorescent groups into azido PEGylated rituximab by click chemistry.
FIG. 4 is an illustration showing the reaction of example 3 using the monomeric moiety of azido PEGylated rituximab in the reaction product of Example 2.
FIG. 5 is an illustration showing the reaction of example 12 using the monomeric moiety of azido PEGylated rituximab in the reaction product of Example 2.
FIG. 6 is a photograph of SDS-PAGE showing the introduction of drugs into azido PEGylated rituximab by click chemistry.
FIG. 7 is a graph showing cell killing effect of various antibodies against Ramos (RA 1) cells and Jurkat cells
FIG. 8 is an SDS-PAGE images showing time-course changes in the transfer reaction of compound 12 to the raw material trastuzumab.
FIG. 9 is a liquid chromatography chart of the reaction solution after a 3-hour transfer reaction.
FIG. 10 is an illustration showing one-pot transfer reaction of compound 12 to native trastuzumab in the reaction of Example 14.

### DESCRIPTION OF EMBODIMENTS

Hereinafter, the present invention is described in detail.

### [1] Sugar compound

The sugar compound of the present invention is a sugar oxazoline derivative and is represented by the following formula (1). 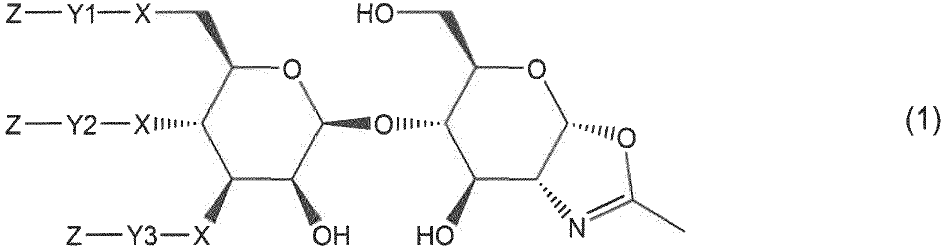 wherein each X is independently a single bond, an oxygen atom, -NH-, -COHN-, -COO-, or a group of formula (5) or (6), 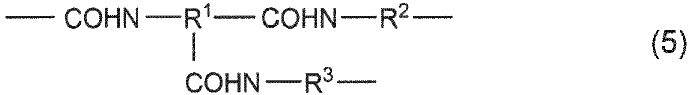 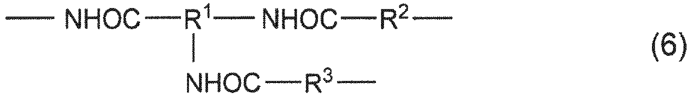 (wherein R¹ is trivalent branched hydrocarbon group having 1 to 6 carbon atoms, R² and R³ are each independently an alkylene group having 1 to 3 carbon atoms)
any one or more of Y1 to Y3 are present and are independently a PEG chain, a substituted PEG chain, or a PEG chain containing an oxygen atom, -NH-, -COHN-, or -COO- in the main chain, a structure of the PEG chain is linear or branched, and the number of branches is 2 to 10 in the case of branched structure,
Z is independently a hydroxy group, a methoxy group, an azido group, a tetrazine group which may be optionally substituted, a norbornene group, a trans-cyclooctene group, a dibenzocyclooctyne group, or a bicyclo[6.1.0]non4-yn-9-ylmethyl group, or a cyanobenzothiazole group which may be optionally substituted, wherein at least one of Z is not a hydroxy group or methoxy group,
when any of Y1 to Y3 is not present, Z is hydrogen and X is oxygen,
when X is the group of formula (5) or (6), Y1 to Y3 are bonded to each of R² and R³ of the branched chains thereof,
when the PEG chain is branched structure, Z is bonded to each of branched chains.
X binds the sugar to the PEG chains or the like, and is independently a single bond, an oxygen atom, -NH-, -COHN-, -COO-, or a group of formula (5) or (6): 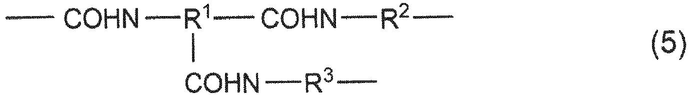 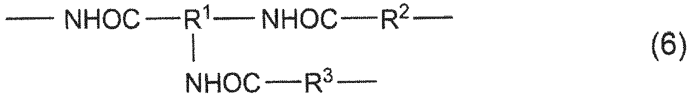 wherein R¹ is trivalent branched hydrocarbon group having 1 to 6 carbon atoms, R² and R³ are each independently an alkylene group having 1 to 3 carbon atoms.
When X is a single bond, an oxygen atom, -NH-, -COHN-, or -COO-, a PEG chain or the like bound to X is one. When X is the group of formula (5) or (6), PEG chain or the like is bonded to each of the branched chains.

Y1 to Y3 are a PEG chain, a substituted PEG chain, or a PEG chain containing an oxygen atom, -NH-, -COHN-, or -COO- in the main chain (hereinafter, collectively sometimes referred to as PEG chains and the like), and a structure of the PEG chain may be linear or branched. PEG chains and the like are non-toxic and non-immunogenic polymers. Protein therapeutics modified with PEG chains and the like can exhibit an effect of enhanced stability in vivo by decreasing immunogenicity, protecting them from degradation by various enzymes, and increasing their half-life.

The number of repeats of the PEG chain is not particularly limited, and it is, for example, 2-1000, 2-100 in some embodiments, 2-50 in some embodiments, 2-30 in some embodiments, 2-20 in some embodiments, 2-10 in some embodiments, 2-5 in some embodiments. The number of repeats is the number of repeats in one PEG chain, and in the case of branched PEG chains, it means the number of repeats in each branched PEG chain. The number of repeats in the after-mentioned PEG chain containing oxygen atom, - NH-, -COHN-, or -COO- in the main chain means the number of repeats in one PEG chain containing these groups.

The substituted PEG chain means a PEG chain in which a hydrogen atom of a side chain of the PEG chain is substituted. The substituents are not particularly limited in the structure that can achieve the effects of the present invention, and there may be mentioned an alkyl group, an aralkyl group, an alkenyl group, an alkynyl group, a hydroxyl group, an amino group, an aldehyde group, a ketone group, a carboxyl group, an amide group, a thiol group, or an ether group. In particularly, an alkyl group having 1 to 3 carbon atoms is preferable.

The PEG chain containing an oxygen atom, -NH-, -COHN-, or -COO- in the main chain has the oxygen atom (ether bond), -NH-, -COHN- (amide bond), or -COO- (ester bond) between repeating units of polyethylene glycol (CH₂CH₂O) in the main chain. Alternatively, it may contain -NH-, -COHN-, or -COO- in place of the oxygen atom (-O-) of the polyethylene glycol (CH₂CH₂O) in the main chain.

The PEG chain has a linear structure or a branched structure. When it has the branched structure, the number of branches is not limited, but preferably 2 to 10. As mentioned above, the degree of polymerization of PEG chains is not particularly limited, but preferably a degree of polymerization of 2 to 10.

In a site where Y1 to Y3 are present of the general formula (1), Z is a hydroxy group, a methoxy group, an azido group, a tetrazine group which may be optionally substituted, a norbornene group, a trans-cyclooctene group, a dibenzocyclooctyne group, or a bicyclo[6.1.0]non-4-yn-9-ylmethyl group, or a cyanobenzothiazole group which may be optionally substituted. In a site where any of Y1 to Y3 is not present, Z is hydrogen and X is oxygen. At least one of Z is not a hydroxy or methoxy group. At least one of Z is an azido group, a tetrazine group which may be optionally substituted, a norbornene group, a trans-cyclooctene group, a dibenzocyclooctyne group, or a bicyclo[6.1.0]non-4-yn-9-ylmethyl group, or a cyanobenzothiazole group which may be optionally substituted. Drugs can be introduced to these groups. That is, at least one of Z is an azido group, a tetrazine group which may be optionally substituted, a norbornene group, a trans-cyclooctene group, a dibenzocyclooctyne group, or a bicyclo[6.1.0]non-4-yn-9-ylmethyl group, or a cyanobenzothiazole group which may be optionally substituted, and the drugs can be efficiently introduced into the sugar compound of the present invention thereby.

The azido group is represented by -N₃.

The above tetrazine group is 1,2,4,5-tetrazine group. In the substituents of the substituted tetrazine group, the hydrogen atoms at positions 3 and/or 6 are substituted. The substituents at position 3 is not limited, but there may be mentioned an alkyl group having 1 to 3 carbon atoms, a pyridinyl group, a bipyridinyl group, -CF₃ group (fluoroalkyl group), - COOCH₃ (ester group), or a phenyl group. Further, the substituents at position 6 is not limited, but there may be mentioned an amino group, an alkylene group having 1 to 3 carbon atoms (e.g., ethylene group), a phenylene group, or a pyridinylene group. Furthermore, the amino group, the alkylene group having 1 to 3 carbon atoms (e.g., ethylene group), the phenylene group, or the pyridinylene group which is the substituent at position 6, may be bonded to Y1 to Y3 via an amide bond, or an amino group. In addition, the tetrazine group, or the substituted tetrazine group, may be bonded directly to the PEG chain or the like., but may also be bonded by, for example, ether bond, ester bond, carbonate bond, carbamate bond, amide bond, or the like.

Specific substituted tetrazine groups are not limited, but include groups represented by the following formulas

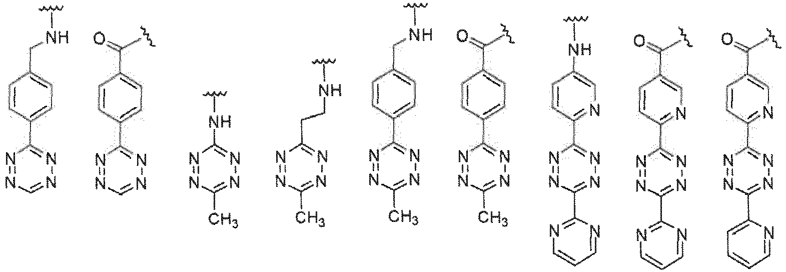

The norbornene group is represented by the following formula (8). 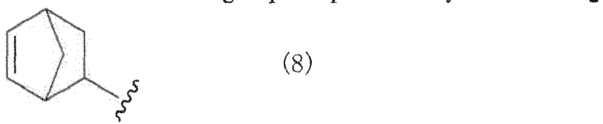 The norbornene group may be directly bonded to the PEG chain or the like, but may also be bonded by, for example, ether bond, ester bond, carbonate bond, carbamate bond, or amide bond.

The trans-cyclooctene group is represented by the following formula (9). 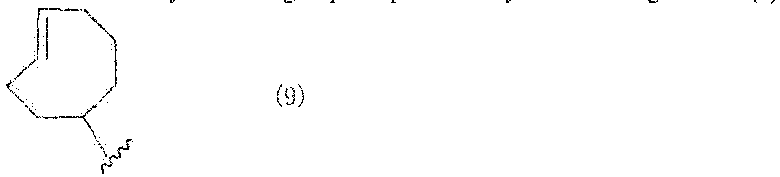 The trans-cyclooctene group may be directly bonded to the PEG chain or the like, but may also be bonded by, for example, ether bond, ester bond, carbonate bond, carbamate bond, or amide bond.

The dibenzocyclooctyne group is represented by the following formula (10). 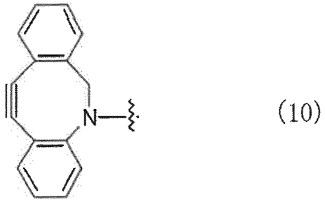 The dibenzocyclooctyne group may be directly bonded to the PEG chain or the like, but may also be bonded by, for example, ether bond, ester bond, carbonate bond, carbamate bond, or amide bond.

The bicyclo[6.1.0]non-4-yn-9-ylmethyl group is represented by the following formula (11). 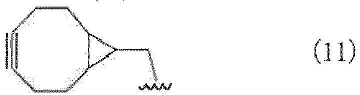 The bicyclo[6.1.0]non-4-yn-9-ylmethyl group may be directly bonded to the PEG chain or the like, but may also be bonded by, for example, ether bond, ester bond, carbonate bond, carbamate bond, or amide bond.

The cyanobenzothiazole group is represented by the following formula (12). 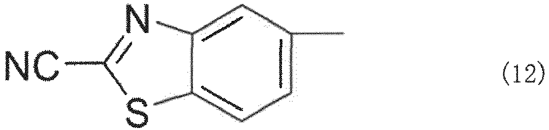 The substituted cyanobenzothiazole group is bonded with Y1 to Y3 via amide bond, ether bond, or ester bond. The cyanobenzothiazole group and the substituted cyanobenzothiazole group may be directly bonded to the PEG chain or the like, but may also be bonded by, for example, ether bond, ester bond, carbonate bond, carbamate bond, or amide bond.

When Y1 to Y3 is a branched structure and Z is two or more in Y1 to Y3, Z may be identical or may not be identical.

The Substituent in Y1 to Y3 of the general formula (1) is not particularly limited, but includes an organic group or a functional group. The organic group is an aggregate of atoms that essentially contain a carbon atom, and for example, there may be mentioned an alkyl group, an aralkyl group, an alkenyl group, and an alkynyl group. In particular, the alkyl group and alkenyl group is preferable. There is no restriction on a structure of the alkyl group and alkenyl group, and they may be saturated or unsaturated chain hydrocarbon groups or alicyclic hydrocarbon groups. The number of carbons in the alkyl group and the alkenyl group is not particularly limited, but preferably 1 to 30, more preferably 1 to 20. The functional group is an aggregate of atoms that determine the properties of organic compounds, and for example, there may be mentioned a hydroxyl group, an amino group, an aldehyde group, a ketone group, a carboxyl group, an amide group, a thiol group, or an ether group. In particular, the hydroxyl group, amino group, carboxyl group, amide group or ether group is preferable.

As the sugar compound (sugar oxazoline derivative) of the present invention, the sugar compound (oxazoline derivative) represented by the following formula (2) is preferable.

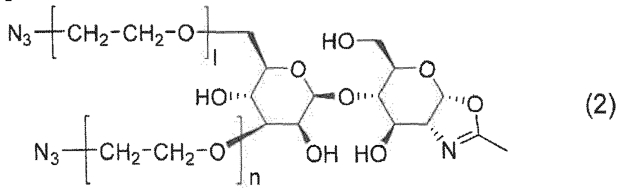

In the general formula (2), the degree of polymerization of polyethylene glycol chain is not particularly limited, but preferably a degree of polymerization of 2 to 10

More specifically, the sugar compound (sugar-oxazoline derivative) of the present invention includes the sugar compounds (oxazoline derivatives) represented by the following formulas.

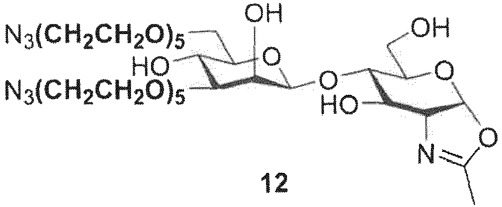

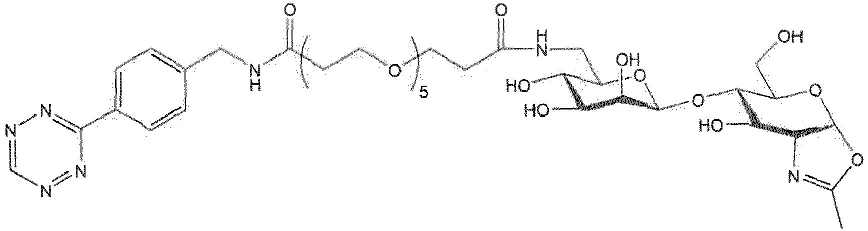

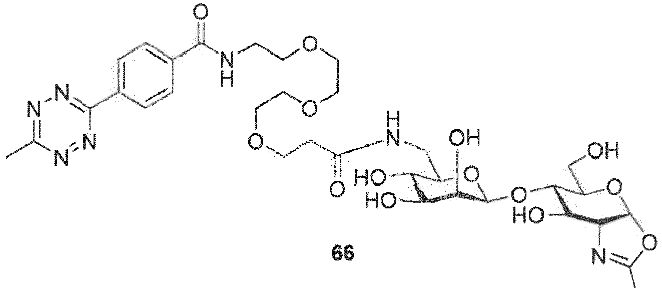

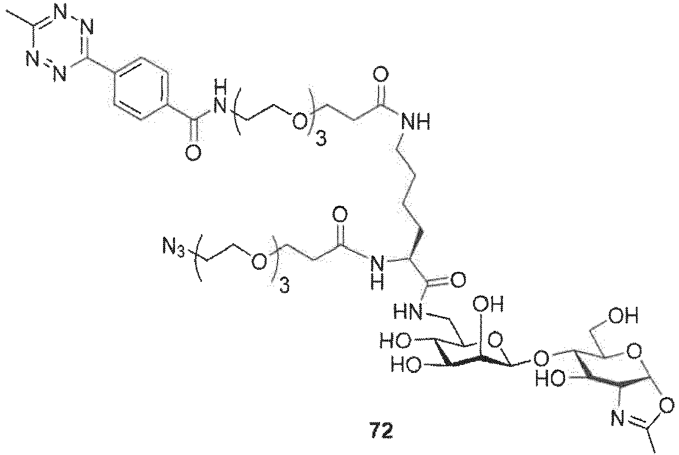

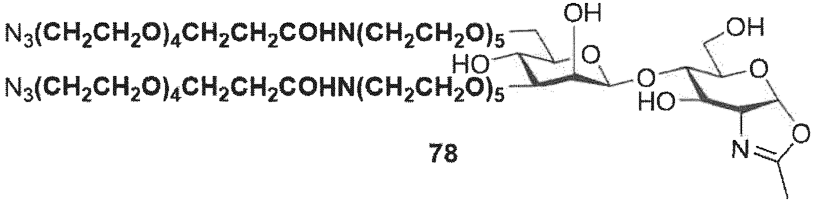

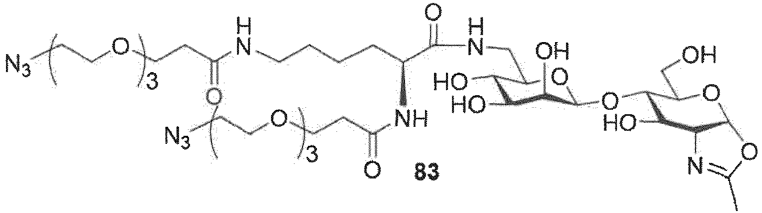

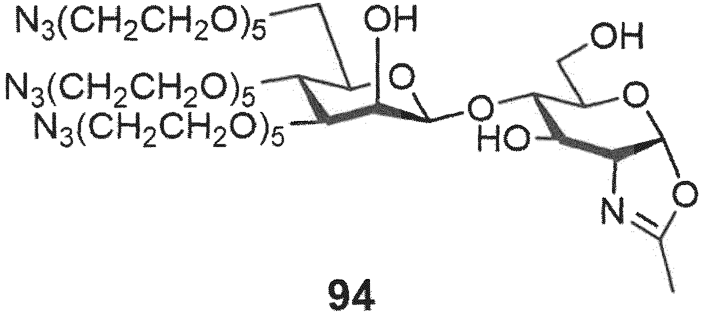

### [2] Modified immunoglobulin antibody

The modified immunoglobulin antibody of the present invention has a sugar compound group having the polyethylene glycol chain of the following formula (7), which is derived from the sugar compound having the polyethylene glycol chains at the sugar-binding site of the side chain of an asparagine residue in the Fc region. 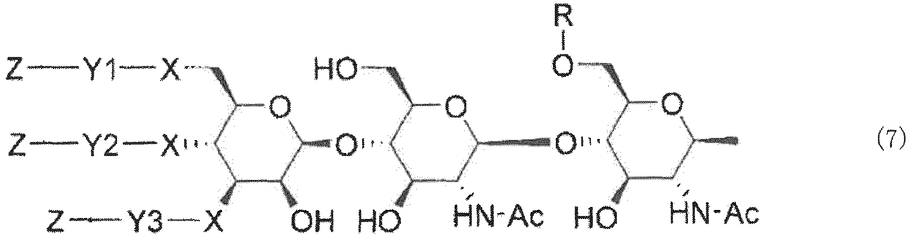 wherein X, Y1 to Y3, and Z are as above mentioned, and R is L-fucose or a hydrogen atom.

The modified immunoglobulin antibody having the sugar compound group represented by the above formula (7) is obtained. by reacting an antibody with the sugar compound (sugar oxazoline derivative) represented by the general formula (1) in the presence of ENGase.

The Antibody is not particularly limited, but there may be mentioned IgG, IgA1, IgA2, IgE, IgD, or IgM. The asparagine residue in the Fc region of IgG is at 297th. The asparagine residues in the Fc region of IgA1 are at 144th and 340th. The asparagine residues in the Fc region of IgA2 are at 131st, 205th, and 327th. The asparagine residues in the Fc region of IgE are at 140th, 168th, 218th, 265th, 371st, 383rd, and 394th. The asparagine residues in the Fc region of IgD are at 354th, 445th, and 496th. The asparagine residues in the Fc region of IgM are at 171st, 332nd, 395th, 402nd, and 563rd.

In the case of IgG, for example, a modified IgG antibody represented by the following general formula (3), which is the ADC precursor, can be obtained. 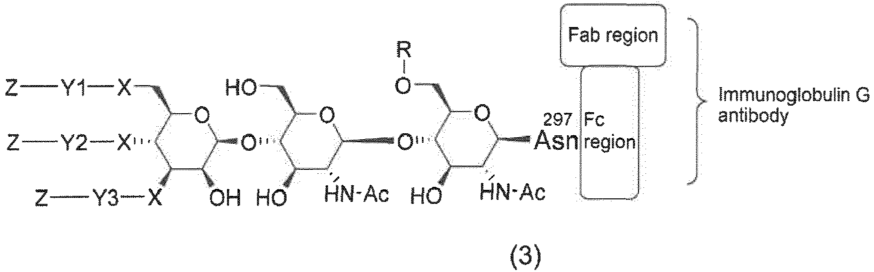 wherein X, Y1 to Y3, and Z are as above mentioned, and R is L-fucose or a hydrogen atom.

Among the modified IgG antibodies represented by the general formula (3) which are ADC precursors, the modified IgG antibody represented by the following general formula (4) is particularly preferable as the product of the present invention. 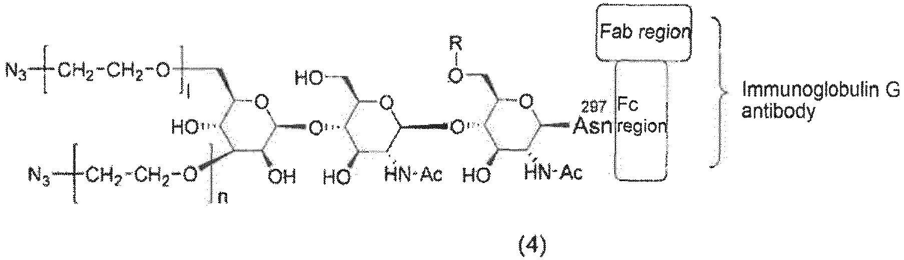 wherein R is L-fucose or a hydrogen atom, and 1 and n are integers of 2 to 10.

### [3] Antibody-drug conjugate

In the antibody-drug conjugate of the present invention, the drug is bound to Z of the sugar compound via, for example, a linker (which is a molecule that connects the drug to the antibody and controls the release rate, release mechanism, and release site of the drug), and the asparagine residue of the antibody is bound to the oxazoline backbone of the sugar compound. The linker is not limited, but include, for example, Val-Cit linker, MCC linker, or Gly-Gly-Phe-Gly linker. The sugar compounds (sugar oxazoline derivatives) of the present invention are introduced into the asparagine of the Fc moiety of the antibody by reacting with the antibody in the presence of ENGase, as described above.

In the modified immunoglobulin antibody of the present invention, the drugs are bound to the Z group. According to the Z groups (azido group, tetrazine group derivative, norbornene group, trans-cyclooctene group, dibenzocyclooctyne group, or bicyclo[6.1.0]non-4-yn-9-ylmethyl group, or cyanobenzothiazole group derivative), drugs can be introduced under mild conditions. For example, the reaction can proceed rapidly at room temperature at a pH of neutral range. Therefore, the antibodies are not exposed to high temperatures, and no degradation of the antibodies occurs.

### EXAMPLES

The present invention now will be further illustrated by, but is by no means limited to, the following Examples.

### <<Example 1>>

In this Example, a sugar compound having a PEG chain with an azide group at the end was prepared.

### (Preparation of compound 2)

Compound 1 (2.03 g, 8.52 mmol) was dissolved in toluene (40 mL), p-methoxybenzylchloride (1.27 mL, 9.37 mmol), potassium iodide (283 mg), silver oxide (I) (2.96 g, 12.8 mmol) were added sequentially, and the mixture was stirred at 60°C overnight. The solid matter was separated by filtration and washed with chloroform, and then the filtrate and washing solution were combined and concentrated under reduced pressure. The resulting residue was purified by silica gel column chromatography (chloroform:methanol = 50:1) to obtain yellow oily compound 2 (1.66 g, 58 %). 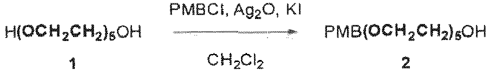 Compound 2 ¹H NMR (600 MHz, CDCl₃):δ=2.66(brs, 1H), 3.57-3.62(m, 4H), 3.63-3.69(m, 14H), 3.69-3.74(m, 2H), 3.80(s, 3H), 4.50(s, 2H), 6.87(d, J=8.9 Hz, 2H), 7.27(d, J=7.1 Hz, 2H).

### (Preparation of compound 3)

Compound 2 (773 mg, 2.28 mmol) was dissolved in dichloromethane (15 mL), triethylamine (1.57 mL, 11.4 mmol) and tosyl chloride (610 mg, 3.20 mmol) were added, and the mixture was stirred at room temperature overnight. Water was added to the reaction solution, and the mixture was extracted twice with chloroform. The organic phase was washed with saturated sodium hydrogen carbonate aqueous solution and saturated saline in this order, and then the mixture was dried with magnesium sulfate. After filtration of the desiccant, the solvent was removed under reduced pressure. The residue was purified by silica gel column chromatography (toluene:ethyl acetate = 2:3) to obtain pale yellow compound 3 (1.08 g, 92 %). 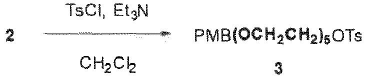 Compound 3 ¹H NMR(600 MHz, CDCl₃):δ=2.44(s, 3H), 3.55-3.71(m, 18H), 3.80(m, 3H), 4.15(t, J=4.8 Hz, 2H), 4.49(s, 2H), 6.87(d, J=8.2Hz, 2H), 7.26(d, J=8.9 Hz, 2H), 7.34(d, J=8.2 Hz, 2H), 7.80(d, J=8.2 Hz, 2H).

### (Preparation of compound 5)

Compound 4 (435 mg, 522 µmol), prepared by the method described in Non-Patent literature 3, and compound 3 (1.07 g, 2.09 mmol) were dissolved in DMF (2.1 mL), sodium hydride (50.1 mg, 1.15 mmol) was added, and the mixture was stirred at 0°C for 10 minutes, and then at room temperature overnight. 5% citric acid aqueous solution (2 mL) was added to the reaction solution to stop the reaction, and then water was added, and the reaction solution was extracted twice with ethyl acetate. The organic phase was washed with saturated sodium hydrogen carbonate aqueous solution and saturated saline in this order, then magnesium sulfate was added for desiccation. After filtration of the desiccant, the solvent was removed under reduced pressure. The residue was purified by silica gel column chromatography (ethyl acetate) to obtain colorless oily compound 5 (624 mg, 79 %). 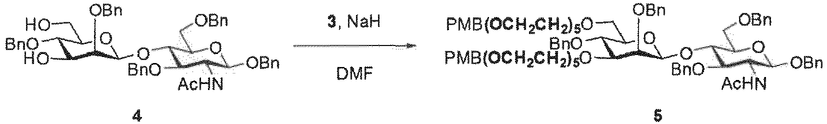 Compound 5 ¹H NMR(600 MHz, CDCl₃):δ=1.66(s, 3H), 3.23-3.30(m, 2H), 3.42-3.47(m, 2H), 3.47-3.87(m, 52H), 3.90(t, J=6.2 Hz, 1H), 4.05(t, J=6.2 Hz, 1H), 4.45(d, J=11.7 Hz, 1H), 4.46-4.50(m, 5H), 4.52-4.60(m, 3H), 4.64(d, J=11.7 Hz, 1H), 4.75-4.91(m, 6H), 6.11(d, J=8.2 Hz, 1H), 6.86(d, J=8.9 Hz, 4H), 7.19-7.34(m, 27H), 7.40(d, J=7.6 Hz, 2H).

### (Preparation of compound 6)

Compound 5 (627 mg, 414 µmol) was dissolved in dichloromethane (6 mL), water (0.6 mL) and DDQ (263 mg, 1.16 mmol) were added, and the mixture was stirred at 0°C for 15 minutes, and then at room temperature for 2.5 hours. Saturated sodium hydrogen carbonate aqueous solution (18 mL) and chloroform (12 mL) were added to the reaction solution, and the mixture was stirred at room temperature for 10 minutes and extracted twice with chloroform. The organic phase was washed with saturated sodium hydrogen carbonate aqueous solution and saturated saline in this order, and then dried with sodium sulfate. After filtration of the desiccant, the solvent was removed under reduced pressure. The residue was purified by silica gel column chromatography (ethyl acetate:methanol = 8:1) to obtain colorless oily compound 6 (286 mg, 54 %). 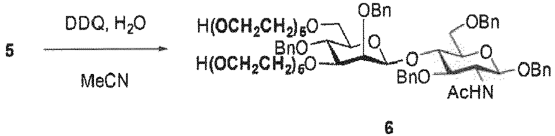 Compound 6 ¹H NMR(600 MHz, CDCl₃):δ=1.68(s, 3H), 2.83(brs, 1H), 2.95(brs, 1H), 3.26-3.31(m, 2H), 3.42-3.84(m, 48H), 3.91(t, J=6.9 Hz, 1H), 4.07(t, J=6.9 Hz, 1H), 4.46(d, J=11.7 Hz, 1H), 4.50(s, 1H), 4.53-4.61(m, 3H), 4.64(d, J=11.7 Hz, 1H), 4.75-4.91(m, 6H), 6.26(d, J=8.2 Hz, 1H), 7.18-7.36(m, 23H), 7.40(d, J=7.6 Hz, 2H).

### (Preparation of compound 7)

Compound 6 (663 mg, 520 µmol) was dissolved in dichloromethane (6 mL), triethylamine (582 µL, 4.16 mmol) and tosyl chloride (397 mg, 2.08 mmol) were added sequentially, and the mixture was stirred at room temperature overnight. Water was added to the reaction solution, and the mixture was extracted twice with ethyl acetate. The organic phase was washed with saturated sodium hydrogen carbonate aqueous solution and saturated saline in this order, and then dried with magnesium sulfate. After filtration of the desiccant, the solvent was removed under reduced pressure. The residue was purified by silica gel column chromatography (toluene:ethyl acetate = 1:20) to obtain colorless oily compound 7 (658 mg, 80%). 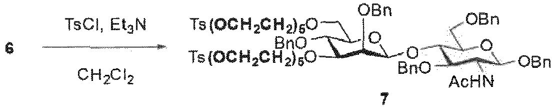 Compound 7 ¹H NMR(600 MHz, CDCl₃):δ=1.66(s, 3H), 2.43(s, 6H), 3.24-3.30(m, 2H), 3.43-3.85(m, 44H), 3.91(t, J=6.2 Hz, 1H), 4.05(t, J=6.2 Hz, 1H), 4.11-4.14(m, 4H), 4.44(d, J=11.7 Hz, 1H), 4.49(s, 1H), 4.52-4.60(m, 3H), 4.63(d, J=11.7 Hz, 1H), 4.75-4.91(m, 6H), 6.08(d, J=8.2 Hz, 1H), 7.17-7.38(m, 27H), 7.40(d, J=6.9Hz, 2H), 7.78(d, J=8.2Hz, 4H).

### (Preparation of compound 8)

Compound 7 (422 mg, 279 µmol) was dissolved in 1,4-dioxane (16 mL), the reaction solution was added to a suspension of Pd/C (260 mg) in 1,4-dioxane (4 mL), then water (5 mL) was added, and the mixture was stirred overnight at room temperature under hydrogen atmosphere. The solid matter was separated by filtration and washed with 50% 1, 4-dioxane aqueous solution, and then the filtrate and washing solution were combined and concentrated under reduced pressure. The resulting residue was purified by silica gel column chromatography (chloroform:methanol:water = 85:15:0.8) to obtain colorless oily compound 8 (245 mg, 78 %). 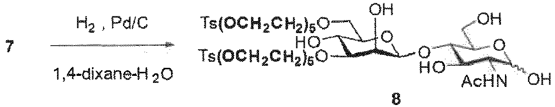 Compound 8 ¹³C NMR(150 MHz, CD₃OD):δ=21.65, 22.70, 23.00, 55.65, 58.27, 58.36, 62.10, 67.58, 69.14, 69.17, 69.79, 69.90, 70.63, 71.03, 71.37, 71.47, 71.52, 71.55, 71.61, 71.82, 71.86, 71.88, 73.91, 76.49, 76.89, 81.62, 82.11, 83.78, 83.80, 92.25, 97.32, 102.07, 102.15, 129.12, 131.13, 146.52, 173.46, 174.03

### (Preparation of compound 9)

Compound 8 (222 mg, 196 µmol) was dissolved in pyridine (2 mL), acetic anhydride (1 mL) was added, and the mixture was stirred overnight at room temperature. Methanol (2mL) was added to the reaction solution to dissolve the excess reagent, and the reaction solution was concentrated under reduced pressure. The residue was purified by silica gel column chromatography (chloroform:methanol:water = 20:1) to obtain colorless oily compound 9 (131 mg, 50%, α/β=7/3). 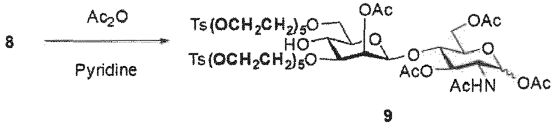 Compound 9 ¹H NMR(600 MHz, CDCl₃):δ=1.93(s, 3H), 2.07(s, 3H), 2.09(s, 0.9H), 2.10-2.13(m, 9H), 2.18(s, 2.1H), 2.45(s, 6H), 3.44-3.77(m, 40H), 3.77-3.83(m, 0.3H), 3.86-3.95(m, 1.7H), 4·13-4.19(m, 4H), 4.22-4.40(m, 3H), 4.55(s, 0.7H), 4.58(s, 0.3H), 4.91-5.01(m, 1H), 5.12(t, J=8.9 Hz, 0.3H), 5.20(dd, J=8.9 Hz, 11.0 Hz, 0.7H), 5.45(d, J=3.4 Hz, 0.3H), 5.48(d, J=3.4 Hz, 0.7H), 5.61(d, J=8.9 Hz, 0.3H), 5.62(d, J=8.9 Hz, 0.7H), 5.88(d, J=9.6 Hz, 0.3H), 6.10(d, J=4.1 Hz, 0.7H), 7.35(d, J=8.2 Hz, 4H), 7.80(d, J=8.2 Hz, 4H).

### (Preparation of compound 10)

Compound 9 (76.2 mg, 56.8 µmol) was dissolved in DMF (1.5 mL), sodium azide (36.9 mg, 568 µmol) was added, and the mixture was stirred at 50°C overnight. Water was added to the reaction solution, and the mixture was extracted twice with ethyl acetate. The organic phase was washed with saturated sodium hydrogen carbonate aqueous solution and saturated saline in this order, and then dried with magnesium sulfate. After filtration of the desiccant, the solvent was removed under reduced pressure. The residue was purified by silica gel column chromatography (chloroform:methanol:water = 30:1) to obtain colorless oily compound 10 (48.9 mg, 80%, α/β=75/25). 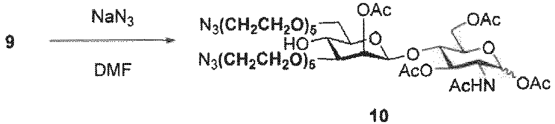 Compound 10 ¹H NMR(600 MHz, CDCl₃):δ=1.93(s, 3H), 2.08(s, 3H), 2.10(s, 0.75H), 2.11-2.14(m, 9H), 2.18(s, 2.25H), 3.39(t, J=4.8 Hz, 4H), 3.44-3.78(m, 40H), 3.78-3.83(m, 0.25H), 3.87-3.95(m, 1.75H), 4.20-4.40(m, 3H), 4.53(s, 0.75H), 4.56(s, 0.25H), 4.92-5.00(m, 1H), 5.11(t, J=9.6 Hz, 0.25H), 5.19(dd, J=8.9 Hz, 11.0 Hz, 0.75H), 5.45(d, J=3.4 Hz, 0.25H), 5.48(d, J=3.4 Hz, 0.75H), 5.56(d, J=8.9 Hz, 0.75H), 5.61(d, J=8.2 Hz, 0.25H), 5.81(d, J=9.6 Hz, 0.25H), 6.11(d, J=4.1Hz, 0.75H).

### (Preparation of compound 11)

Compound 10 (41.3 mg, 38.1 µmol) was dissolved in methanol (10 mL), and a catalyst amount of 28% sodium methoxide methanol solution was added dropwise and the mixture was stirred overnight at room temperature. Amberlite IR-120 (H⁺ form) was added to the reaction solution to neutralize it, and then the resin was separated by filtration. The filtrate was concentrated under reduced pressure, and the residue was purified by silica gel column chromatography (chloroform:methanol:water = 85:15:0.1) to obtain colorless oily compound 11 (14.6 mg, 44 %). 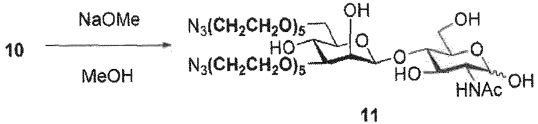 Compound 11 ¹H NMR(600 MHz,CD₃OD):δ=1.93(s,3H),3.37-3.89(m,51H),4.18-4.23(m,1H),4.62-4.67(m,1.4H),5.12(d,J=2.7 Hz,0.6H).

### (Preparation of compound 12)

0.780 M 2-chloro-1,3-dimethylimidazolinium chloride aqueous solution (300 µL, 234 µmol) and triethylamine (82.1 µL, 589 µmol) were added to compound 11 (6.6 mg, 7.55 µmol) and the mixture was stirred at 0°C overnight. The reaction solution was purified by gel filtration chromatography (0.05% triethylamine solution) to obtain compound 12 quantitatively (6.57 mg). 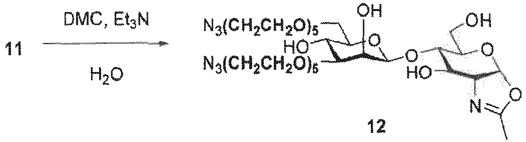 Compound 12 ¹H NMR(600 MHz, D₂O):δ=1.93(s, 3H), 3.23-3.28(m, 1H), 3.30(dd, J=2.7 Hz, 9.6 Hz, 1H), 3.32-3.40(m, 6H), 3.46-3.78(m, 41H), 4.01-4.07(m, 2H), 4.22(s, 1H), 4.55(s, 1H), 5.93(d, J=7.5 Hz, 1H).

### «Example 2»

In this Example, the compound obtained in Example 1 was conjugated to rituximab (antibody).

### (Transfer reaction of compound 12 to the sugar chain binding site of rituximab)

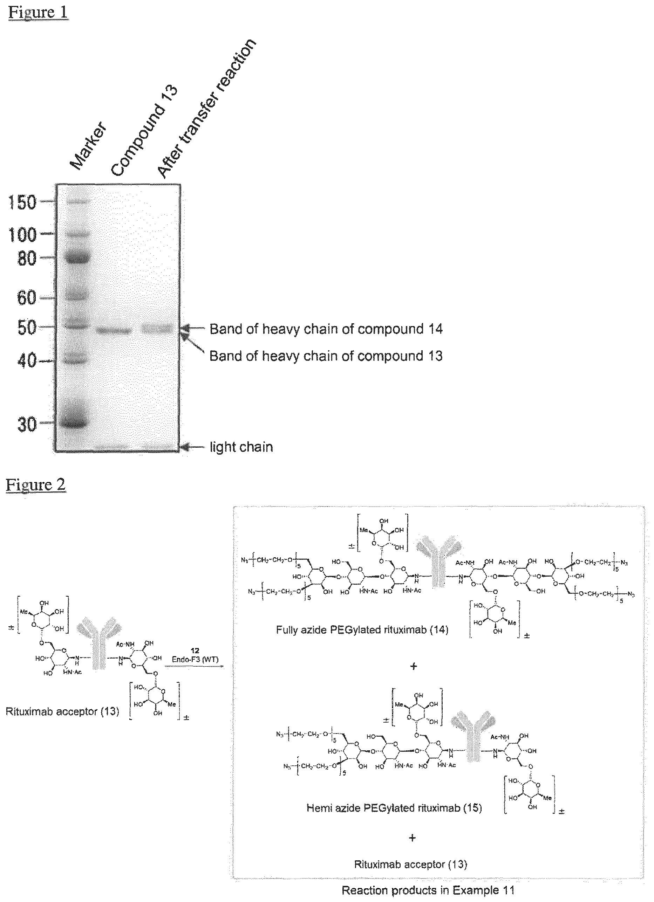

The Rituximab acceptor (13) was prepared according to Non-Patent literature 4 using rituximab manufactured by Zenyaku Kogyo Co., Ltd. For the transfer reaction of compound 12 to the sugar chain binding site of rituximab acceptor 13, the prepared rituximab acceptor 13 (2 mg), compound 12 (1.384 µmol), and wild-type enzyme Endo-F3 WT (200 µg) expressed and purified in E. coli were added in 50mM Tris-HCl buffer solution (pH 7.0) in a total volume of 0.5 mL and incubated at 37°C for 1 hour. A portion of the reaction solution was removed and confirmed by SDS-PAGE (Coomassie Brilliant Blue staining) using a 9.0% polyacrylamide gel, and a heavy chain band shifted to the high molecular weight side by the transfer of compound 12 was confirmed (Figure 1). 80 µL of Ab-Capcher ExTra (ProteNova Co., Ltd.) equilibrated with the same buffer solution was added to the reaction solution in wet volume, and the mixture was shaken by rotation for 1 hour at room temperature to adsorb rituximab on the gel carrier. The gel carrier was washed three times with 1.8 mL of NETN buffer solution (50mM Tris-HCl buffer solution (pH8.0), 150mM sodium chloride, 1mM EDTA, and 0.1% (w/v) NP-40) by inversion mixing at room temperature, washed with 1.8 mL of NETN buffer solution for 5 minutes at room temperature by rotary shaking, and rinsed with 1 mL of PBS four times. Rituximab was eluted by adding 200 µL of 0.1M glycine hydrochloride buffer solution (pH 2.7) to the washed carrier, and 6.67 µL of 1M Tris-HCl buffer solution (pH 9.0) was added to the eluate to neutralize it. The elution procedure was performed four times in total, and the eluate was concentrated with Amicon Ultra-0.5 (cut-off molecular weight: 30 kDa, Merck Millipore) and replaced with PBS. As a result, 1.46 mg of a mixture of Fully azide PEGylated rituximab body (14), Hemi azide PEGylated rituximab body (15), and unreacted rituximab acceptor (13) was obtained as the product of the transfer of compound 12 to the sugar chain binding site of rituximab (Figure 2). Mass spectrometry analysis showed that the transfer yield of compound 12 to the sugar chain moiety of the heavy chain was 46.7%.

### «Example 3»

In this Example, a fluorescent group was introduced into the azide group.

### (Introduction of fluorescent group into azido PEGylated rituximab)

To 4.0 µL of the aqueous solution of the reaction product obtained in Example 2 (7.0 µg/µL), 2.0 µL of 1.0 M phosphate buffer (pH 8.0), 15.3 µL of water, and 18.7 µL of 1% DMSO aqueous solution of compound 16 (100 µM) were added, and the mixture was shielded from light and allowed to react at room temperature for 2 hours.

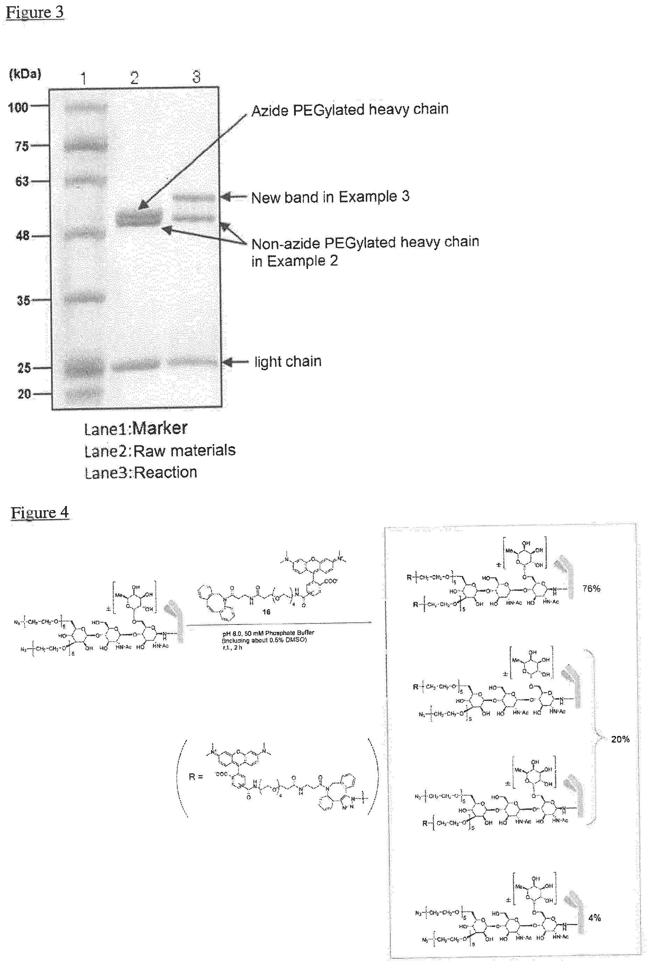

A portion (3.3 µL) of the reaction solution was removed and examined by SDS-PAGE (Coomassie Brilliant Blue staining) using a 10% polyacrylamide gel, and it was confirmed that the band corresponding to the azido PEGylated heavy chain in the material was shifted to the high molecular weight side by the click reaction and that a fluorescent group was introduced (Figure 3).

Mass spectrometry analysis showed that 76% of the products had both fluorescent groups introduced to the two azide groups on the heavy chains, 20% had only one of the two fluorescent groups introduced, and 4% were unreacted (Figure 4).

### «Example 4»

In this Example, a sugar compound having a PEG chain with a tetrazine group at the end was prepared.

### (Preparation of compound 18)

Commercially available compound 17 (5.01 g, 11.2 mmol) was dissolved in dichloromethane (6 mL), 25% hydrogen bromide acetic acid solution (15 mL) was added, and the mixture was stirred at room temperature for 2.5 hours. Ice water was added to the reaction solution, and the mixture was extracted with chloroform. The organic phase was washed with water (twice), saturated sodium hydrogen carbonate aqueous solution and saturated saline were added in this order, and then dried with magnesium sulfate. After filtration of the desiccant, the solvent was removed under reduced pressure. The resulting residue (4.96 g) was dissolved in benzyl alcohol (14 mL) and stirred at 90°C overnight. Acetone (40 mL), water (15 mL), and hydrochloric acid (10mL) were added to the reaction solution, and the mixture was stirred at 75°C for another 5 hours. Triethylamine (20 mL) was added to the reaction solution to stop the reaction, and the mixture was concentrated under reduced pressure. Water was added to the residue and the mixture was extracted with ethyl acetate (twice). The organic phase was washed with saturated sodium hydrogen carbonate aqueous solution and saturated saline in this order, and then dried with magnesium sulfate. After filtration of the desiccant, the solvent was removed under reduced pressure. The residue was purified by silica gel column chromatography (CHCl₃:MeOH = 10:1) to obtain compound 18 (3.05 g, 68%). 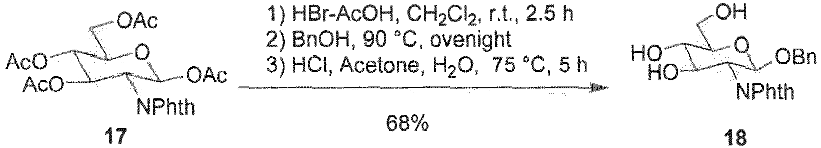 Compound 18 ¹H NMR(600 MHz, CDCl₃):δ=7.82-7.75(m, 2H), 7.73-7.69(m, 2H), 7.14-7.03(m, 5H), 5.25(d, J=8.2 Hz, 1H), 4.80(d, J=12.4 Hz, 1H), 4.53(d, J=12.4 Hz, 1H), 4.36-4.30(m, 1H), 4.16(dd, J=8.2 Hz, 11.0 Hz, 1H), 3.97-3.86(m, 2H), 3.70(dtJ=2.7 Hz, 8.9 Hz, 1H), 3.52-3.48(m, 2H), 3.17(d, J=4.8 Hz, 1H), 2.59(t, J=6.2 Hz, 1H).

### (Preparation of compound 19)

Compound 18 (3.03 g, 7.59 mmol) was dissolved in acetonitrile (125 mL), and benzaldehyde dimethyl acetal (2.27 mL, 15.2 mmol) and CSA (250 mg) were added sequentially, and the mixture was stirred at room temperature overnight. Triethylamine (0.5mL) was added to the reaction solution, and the mixture was concentrated under reduced pressure. Water was added to the residue and the mixture was extracted with ethyl acetate (twice). The organic phase was washed with saturated sodium hydrogen carbonate aqueous solution and saturated saline in this order, and then dried with magnesium sulfate. After filtration of the desiccant, the solvent was removed under reduced pressure. The residue was purified by silica gel chromatography (n-hexane:ethyl acetate = 2:1) to obtain compound 19 (3.36 g, 91%). 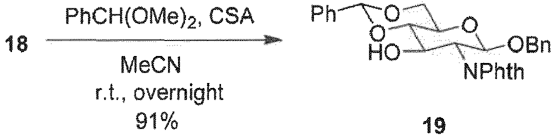 Compound 19 ¹H NMR(600 MHz, CDCl₃):δ=7.86-7.70(m, 4H), 7.53-7.48(m, 2H), 7.41-7.35(m, 3H), 7.11-7.02(m, 5H), 5.58(s, 1H), 5.28(d, J=8.2 Hz, 1H), 4.85(d, J=12.4 Hz, 1H), 4.69-4.60(m, 1H), 4.52(d, J=12.4 Hz, 1H), 4.43(dd, J=3.4 Hz, 9.6 Hz, 1H), 4.30(dd, J=8.9 Hz, 10.3 Hz, 1H), 3.87(t, J=9.6 Hz, 1H), 3.69-3.61(m, 2H), 2.46(d, J=3.4 Hz, 1H).

### (Preparation of compound 20)

Compound 19 (4.36 g, 8.96 mmol) was dissolved in DMF (40 mL), benzyl bromide (5.32 mL, 44.8 mmol) and sodium hydride (589 mg, 13.4 mmol) were added sequentially, and the mixture was stirred at 0°C for 30 minutes and stirred at room temperature overnight. 5% citric acid aqueous solution (5mL) was added to the reaction solution to stop the reaction, then water was added to the reaction solution, and the mixture was extracted with ethyl acetate (twice). The organic phase was washed with saturated sodium hydrogen carbonate aqueous solution and saturated saline in this order, and then dried with magnesium sulfate. After filtration of the desiccant, the solvent was removed under reduced pressure. The residue was purified by silica gel chromatography (toluene:ethyl acetate = 20:1) to obtain compound 20 (4.14 g, 80%). 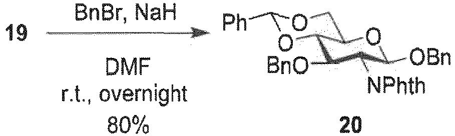 Compound 20¹HNMR(600 MHz, CDCl₃):δ=7.88-7.64(m, 3H), 7.54-7.48(m, 3H), 7.43-7.36(m, 3H), 7.07-6.94(m, 7H), 6.92-6.83(m, 3H), 5.63(s, 1H), 5.19(d, J=8.2 Hz, 1H), 4.80(d, J=12.4 Hz, 1H), 4.77(d, J=12.4 Hz, 1H), 4.48(d, J=12.4 Hz, 2H), 4.45-4.36(m, 2H), 4.26(dd, J=8.9 Hz, 10.3 Hz, 1H), 3.88(t, J=10.3 Hz, 1H), 3.83(t, J=8.9 Hz, 1H), 3.67(m, 1H).

### (Preparation of compound 21)

Compound 20 (4.05 g, 7.01 mmol) was dissolved in dichloromethane (120 mL), triethylsilane (3.34 mL, 21.0 mmol) and boron trifluoride diethyl ether complex (2.91 mL, 23.1 mmol) were added sequentially under argon atmosphere, and then the mixture was stirred at -78°C for 10 min and at 0°C overnight. A mixture of methanol (13mL), triethylamine (6.5mL) and chloroform (19.5mL) was added to the reaction solution to stop the reaction, then saturated sodium hydrogen carbonate aqueous solution was added, and the mixture was extracted with chloroform (twice). The organic phase was washed with saturated saline, and then dried with magnesium sulfate. After filtration of the desiccant, the solvent was removed under reduced pressure. The residue was purified by silica gel chromatography (n-hexane:ethyl acetate = 2:1) to obtain compound 8 (2.98g, 73%). 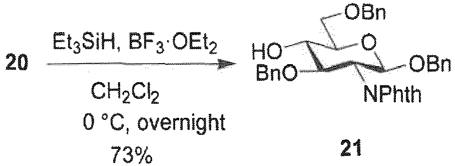 Compound 21 ¹H NMR(600 MHz, CDCl₃):δ=7.85-7.51(m, 4H), 7.41-7.35(m, 4H), 7.35-7.30(m, 1H), 7.11-7.01(m, 7H), 6.99-6.90(m, 3H), 5.15(d, J=7.6 Hz, 1H), 4.78(d, J=12.4 Hz, 1H), 4.72(d, J=12.4 Hz, 1H), 4.67(d, J=12.4 Hz, 1H), 4.60(d, J=12.4 Hz, 1H), 4.51(d, J=12.4 Hz, 1H), 4.47(d, J=12.4 Hz, 1H), 4.26-4.19(m, 2H), 3.89-3.78(m, 3H), 3.68-3.61(m, 1H), 2.89(d, J=2.1 Hz, 1H).

### (Preparation of compound 23)

Commercially available compound 22 (5.65 g, 14.5 mmol) was dissolved in dichloromethane (56 mL), 5-tert-butyl-2-methylbenzenethiol (5.27 mL, 28.9 mmol) and boron trifluoride diethyl ether complex (3.37 mL, 28.9 mmol) were added sequentially, and the mixture was stirred overnight at room temperature. Saturated saline was added to the reaction solution, and the mixture was extracted with chloroform (twice). The organic phase was washed with saturated sodium hydrogen carbonate aqueous solution, and then dried with magnesium sulfate. After filtration of the desiccant, the solvent was removed under reduced pressure. The residue was purified by silica gel chromatography (n-hexane:ethyl acetate = 2:1) to obtain compound 23 (6.75 g, 91%). 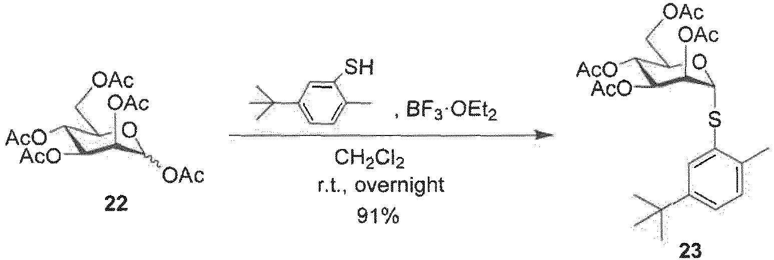 Compound 23 ¹H NMR(600 MHz, CDCl₃):δ=7.53(d, J=2.1 Hz, 1H), 7.23(dd, J=2.1 Hz, 8.2 Hz, 1H), 7.14(d, J=7.6 Hz, 1H), 5.53(s, 1H), 5.43(s, 1H), 5.41-5.34(m, 2H), 4.58-4.49(m, 1H), 4.35(dd, J=4.8 Hz, 12.4 Hz, 1H), 4.08(dd, J=2.7 Hz, 12.4 Hz, 1H), 2.41(s, 3H), 2.17(s, 3H), 2.08(s, 3H), 2.06(s, 3H), 2.03(s, 3H), 1.30(s, 9H).

### (Preparation of compound 24)

Compound 23 (8.07 g, 15.8 mmol) was dissolved in methanol (80 mL), a catalyst amount of 28% sodium methoxide methanol solution was added, and the mixture was stirred overnight at room temperature. Amberlite IR-120 (H⁺ form) was added to the reaction solution to neutralize it, and then the resin was separated by filtration. The filtrate was concentrated under reduced pressure, and the residue was purified by silica gel chromatography (CHCl₃:MeOH = 10:1) to obtain compound 24 (5.29 g, 98%). 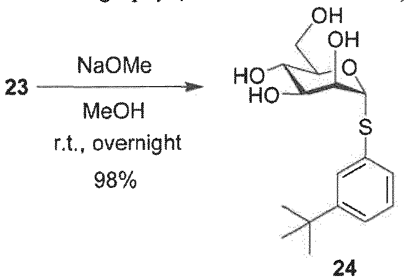 Compound 24 ¹H NMR(600 MHz, CD₃OD):δ=7.60(d, J

### (Preparation of compound 25)

Compound 24 (5.01 g, 14.6 mmol) was dissolved in acetonitrile (250 mL), benzaldehyde dimethyl acetal (3.29 mL, 21.9 mmol) and CSA (507 mg) were added sequentially, and the mixture was stirred for 1 hour at room temperature. Triethylamine (1.5 mL) was added to the reaction solution, and the mixture was concentrated under reduced pressure. The residue was purified by silica gel chromatography (toluene:ethyl acetate = 3:1) to obtain compound 25 (4.12 g, 67%) 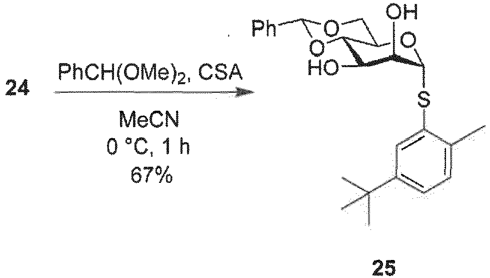 Compound 25 ¹H NMR(600 MHz, CDCl₃):δ=7.58-7.48(m, 3H), 7.42-7.35(m, 3H), 7.25-7.20(m, 1H), 7.16(d, J=7.6 Hz, 1H), 5.59(s, 1H), 5.52(s, 1H), 4.41-4.33(m, 2H), 4.24-4.17(m, 2H), 4.02(t, J=9.6 Hz, 1H), 3.85(t, J=9.6 Hz, 1H), 2.85(s, 1H), 2.77(d, J=3.4 Hz, 1H), 2.41(s, 3H), 1.30(s, 9H).

### (Preparation of compound 26)

Compound 25 (4.08 g, 9.48 mmol) was dissolved in DMF (40 mL), sodium hydride (996 mg, 22.7 mmol) and benzyl bromide (2.70 mL, 22.7 mmol) were added sequentially, and the mixture was stirred at 0°C for 15 minutes and stirred at room temperature overnight. Methanol (3 mL) was added to the reaction solution to dissolve the excess reagent, and then water was added to the reaction solution, and the mixture was extracted with ethyl acetate (twice). The organic phase was washed with saturated sodium hydrogen carbonate aqueous solution and saturated saline in this order, and then dried with magnesium sulfate. After filtration of the desiccant, the solvent was removed under reduced pressure. The residue was purified by silica gel chromatography (Toluene) to obtain compound 26 (4.46 g, 77%). 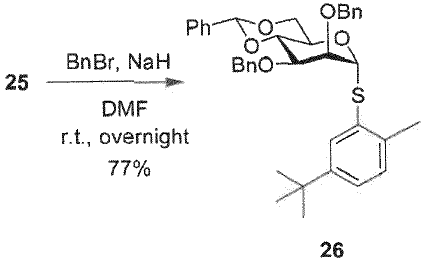 Compound 26 ¹H NMR(600 MHz, CDCl₃):δ=7.52(d, J=6.9 Hz, 2H), 7.48(d, J=2.1 Hz1H), 7.43-7.27(m, 13H), 7.22(dd, J=2.1 Hz, 8.2 Hz, 1H), 7.13(d, J=8.2 Hz, 1H), 5.67(s, 1H), 5.42(s, 1H), 4.84(d, J=12.4 Hz, 1H), 4.74(s, 2H), 4.67(d, J=12.4 Hz, 1H), 4.36-4.30(m, 2H), 4.22-4.19(m, 1H), 4.08(d, J=3.4 Hz, 1H), 4.02(dd, J=3.4 Hz, 9.6 Hz, 1H), 3.91(t, J=9.6 Hz, 1H), 2.31(s, 3H), 1.29(s, 9H).

### (Preparation of compound 27)

Compound 26 (2.17 g, 3.55 mmol), BSP (818 mg, 3.91 mmol) and TTBP (1.77 g, 7.11 mmol) were dissolved in dichloromethane (36 mL), Molecular Sieves 3A (4.11 g) was added under argon atmosphere, and the mixture was stirred at -60°C for 15 minutes. Tf₂O (717 mL) was added, the mixture was stirred for 5 minutes, and then a solution of compound 21 (1.65 g, 2.84 mmol) in dichloromethane (36 mL) was added. The reaction solution was stirred at -60°C for 1 hour, and then the temperature was raised to room temperature over 1.5 hours. Triethylamine (1.2 mL) was added to the reaction solution to stop the reaction, and the solid matter was separated by silica gel filtration and washed with ethyl acetate. The filtrate was combined with the washing solution and concentrated under reduced pressure. The residue was purified by silica gel chromatography (toluene:ethyl acetate = 10:1) to obtain compound 27 (1.83 g, 64%). 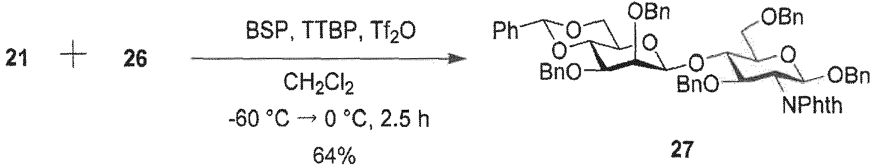 Compound 27 ¹H NMR(600 MHz, CDCl₃):δ=7.83-7.49(m, 4H), 7.49-7.42(m, 4H), 7.39-7.21(m, 16H), 7.11-7.03(m, 5H), 6.93-6.89(m, 2H), 6.83-6.80(m, 3H), 5.51(s, 1H), 5.11(d, J=8.2 Hz, 1H), 4.89-4.82(m, 3H), 4.80(d, J=12.4 Hz, 1H), 4.74(d, J=12.4 Hz, 1H), 4.68(d, J=12.4 Hz, 1H), 4.58(d, J=12.4 Hz, 1H), 4.54(s, 1H), 4.49(d, J=12.4 Hz, 1H), 4.41(d, J=12.4 Hz, 1H), 4.40(d, J=11.0 Hz, 1H), 4.26-4.15(m, 3H), 4.07(t, J=9.6 Hz, 1H), 4.03(t, J=9.6 Hz, 1H), 3.74(d, J=2.7 Hz, 1H), 3.67(dd, J=1.4 Hz, 11.0 Hz, 1H), 3.59-3.55(m, 2H), 3.49-3.46(m, 1H), 3.41(dd, J=3.4 Hz, 10.3 Hz, 1H), 3.14(dt, J=4.8 Hz, 9.6 Hz, 1H), .

### (Preparation of compound 28)

THF solution of 1.0 M BH₃ (12.8 mL, 12.8 mmol) and a dichloromethane solution of 1.0 M n-Bu₂BOTf (2.56 mL, 2.56 mmol) were added to compound 27 (1.72 g, 1.70 mmol) sequentially, and the mixture was stirred at 0°C for 1.5 hours. Triethylamine (1.5 mL) was added to the reaction solution to stop the reaction, and the mixture was concentrated under reduced pressure. The residue was purified by silica gel chromatography (toluene:ethyl acetate = 5:1) to obtain compound 28 (1.53 g, 89%). 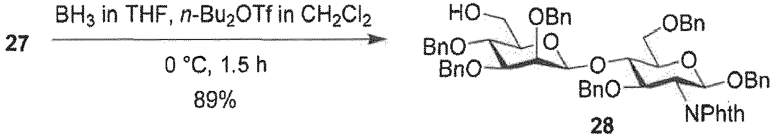 Compound 28 ¹H NMR(600 MHz, CDCl₃):δ=7.83-7.52(m, 4H), 7.44-7.40(m, 2H), 7.35-7.23(m, 18H), 7.11-7.02(m, 5H), 6.90-6.81(m, 5H), 5.13(d, J=8.2 Hz, 1H), 4.90-4.82(m, 4H), 4.80(d, J=12.4 Hz, 1H), 4.67(d, J=11.7 Hz, 1H), 4.56(d, J=11.0 Hz, 1H), 4.53-4.45(m, 5H), 4.40(d, J=12.4 Hz, 1H), 4.27-4.19(m, 2H), 4.00(t, J=9.6 Hz, 1H), 3.79-3.70(m, 4H), 3.66(dd, J=3.4 Hz, 11.0 Hz, 1H), 3.58-3.54(m, 1H), 3.47-3.41(m, 1H), 3.36(dd, J=3.4 Hz, 9.6 Hz, 1H), 3.22-3.18(m, 1H), 2.10(brs, 1H).

### (Preparation of compound 29)

Compound 28 (790 mg, 782 µmol) was dissolved in 1,2-dichloroethane (8 mL), triethylamine (437 µL, 3.13 mmol) and mesylchloride (121 µL, 1.56 mmol) were added sequentially, and the mixture was stirred at 60°C overnight. Water was added to the reaction solution, and the mixture was extracted with ethyl acetate (twice). The organic phase was washed with s saturated sodium hydrogen carbonate aqueous solution and saturated saline in this order, and then dried with magnesium sulfate. After filtration of the desiccant, the solvent was removed under reduced pressure. The residue was purified by silica gel chromatography (toluene:ethyl acetate = 10:1) to obtain c compound 29 (720 mg, 85%). 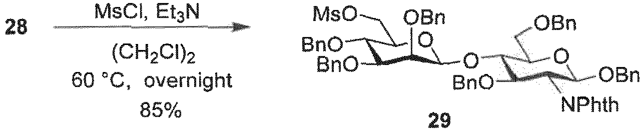 Compound 29 ¹H NMR(600 MHz, CDCl₃):δ=7.83-7.52(m, 4H), 7.41-7.21(m, 20H), 7.13-7.08(m, 1H), 7.06(d, J=4.1 Hz, 4H), 6.84-6.75(m, 5H), 5.14(d, J=8.2 Hz, 1H), 4.91(d, J=11.0 Hz, 1H), 4.90(d, J=12.4 Hz, 1H), 4, 83(s, 2H), 4.79(d, J=12.4 Hz, 1H), 4.71(d, J=11.7 Hz, 1H), 4.58(d, J=11.0 Hz, 1H), 4.55(s, 1H), 4.50(d, J=12.4 Hz, 1H), 4.49-4.43(m, 3H), 4.37(d, J=13.1 Hz, 2H), 4.25-4.18(m, 2H), 4.14(dd, J=8.2 Hz, 10.3 Hz, 1H), 4.02(t, J

### (Preparation of compound 30)

Compound 29 (956 mg, 877 µmol) was dissolved in DMF (9.5 mL), sodium azide (285 mg, 4.38 mmol) was added, and the mixture was stirred at 80°C overnight. Water was added to the reaction solution, and the mixture was extracted with ethyl acetate (twice). The organic phase was washed with saturated sodium hydrogen carbonate aqueous solution and saturated saline in this order, and then dried with magnesium sulfate. After filtration of the desiccant, the solvent was removed under reduced pressure. The residue was purified by silica gel chromatography (toluene:ethyl acetate = 10:1) to obtain compound 30 (862 mg, 95%). 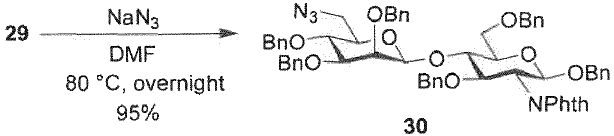 Compound 30 ¹H NMR(600 MHz, CDCl₃):δ=7.81-7.60(m, 3H), 7.46-7.43(m, 3H), 7.35-7.23(m, 18H), 7.10-7.01(m, 5H), 6.91-6.88(m, 2H), 6.77-6.74(m, 3H), 5.11(d, J=8.2 Hz, 1H), 4.91(d, J=11.7 Hz, 3H), 4.86(d, J=12.4 Hz, 1H), 4.78(d, J=12.4 Hz, 1H), 4.67(d, J=12.4 Hz, 1H), 4.56(d, J=11.0 Hz, 1H), 4.56(s, 1H), 4.51-4.41(m, 5H), 4.26-4.19(m, 2H), 4.05(dd, J=8.2 Hz, 9.6 Hz, 1H), 3.82(t, J=9.6 Hz, 1H), 3.77(d, J=2.7 Hz, 1H), 3.72(dd, J=2.1 Hz, 11.0 Hz, 1H), 3.65(dd, J=3.4 Hz, 11.0 Hz, 1H), 3.55-3.51(m, 1H), 3.45(d, J=12.4 Hz, 1H), 3.32(dd, J=3.4 Hz, 9.6 Hz, 1H), 3.27-3.24(m, 1H), 3.22(dd, J=5.5 Hz, 12.4 Hz, 1H).

### (Preparation of compound 31)

Compound 30 (156 mg, 150 µmol) was dissolved in a mixture of methanol (4 mL)-THF (1 mL), ethylenediamine (1 mL) was added, and the mixture was heated to reflux overnight. The reaction solution was concentrated under reduced pressure, pyridine (1 mL) and acetic anhydride (2 mL) were added to the residue, and the mixture was stirred overnight at room temperature. Methanol (2 mL) was added to the reaction solution to stop the reaction, and the mixture was concentrated under reduced pressure. Water was added to the residue, and the mixture was extracted with ethyl acetate. The organic phase was washed with 1M hydrochloric acid, saturated sodium hydrogen carbonate aqueous solution, and saturated saline in this order, and then dried with magnesium sulfate. After filtration of the desiccant, the solvent was removed under reduced pressure. The residue was purified by silica gel chromatography (toluene:ethyl acetate = 5:1) to obtain compound 31 (137 mg, 96%). 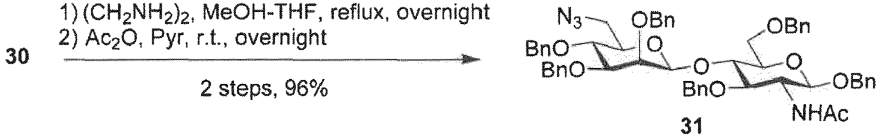 Compound 31 ¹H NMR(600 MHz, CDCl₃):δ=7.

### (Preparation of compound 32)

Compound 31 (101 mg, 103 µmol) was dissolved in 1,4-dioxane (10.0 mL), and the reaction solution was added to a suspension of palladium hydroxide (99.5 mg) in 1,4-dioxane (2.0mL). Water (3.0 mL) and 1N hydrochloric acid (155 µL, 155 µmol) were added, and the mixture was stirred at room temperature for 4 hours under a hydrogen atmosphere. The solid matter was separated by filtration, washed with water, and the filtrate was combined with the washing solution and lyophilized to obtain compound 32 (42.9 mg). 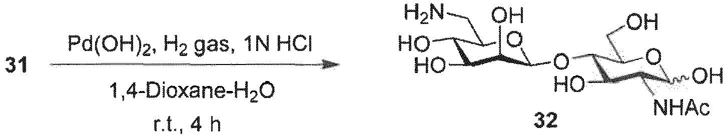 Compound 32 ¹H NMR(600 MHz, CDCl₃):δ=7.39(d, J=6, 9 Hz, 2H), 7.33-7.21(m, 28H), 5.80(d, J=7.6 Hz, 1H), 4.92-4.86(m, 4H), 4.84-4.79(m, 2H), 4.63-4.55(m, 4H), 4.53-4.44(m, 4H), 4.03(t, J=7.6 Hz, 1H), 3.89(t, J=6.9 Hz, 1H), 3.79-3.75(m, 2H), 3.70-3.61(m, 4H), 3.38(dd, J=2.7 Hz, 8.9 Hz, 1H), 3.09-3.05(m, 1H), 2.92(dd, J=2.7 Hz, 13.1 Hz, 1H), 2.61(dd, J=7.5 Hz, 13.1 Hz, 1H), 1.69(s, 3H), 1.48(brs, 2H).

### (Preparation of compound 34)

Compound 32 (42.9 mg) was dissolved in acetonitrile aqueous solution (0.80mL), triethylamine (6.96 µL, 50.0 µmol) and compound 33 (15.1 mg, 25.0 µmol) were added sequentially, and the mixture was stirred at room temperature for two hours. Water (2.0 mL) was added to the reaction solution, and the mixture was lyophilized. The residue was purified by silica gel column chromatography (chloroform:methanol:water =7.5:2.5:0.3) to obtain compound 34 (22.2 mg, 99 % in 2 steps). 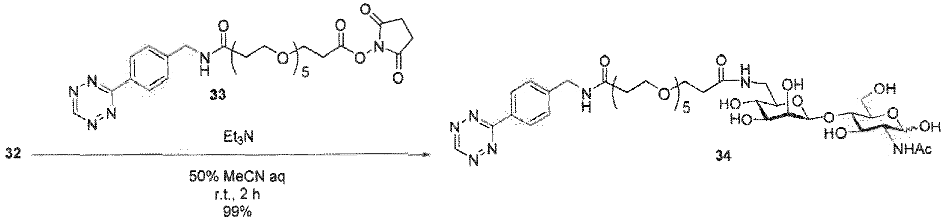 Compound 34¹H-NMR(600 MHz, D₂O):δ=10.35(s, 1H), 8.55(d, J=8.2 Hz, 2H), 7.58(d, J=8.2 Hz, 2H), 5.10(d, J=2.1 Hz, 0.8H), 4.64(s, 1H), 4.58(d, J=8.2 Hz, 0.2H), 4.53(s, 2H), 3.93-3.56(m, 28H), 3.49-3.43(m, 3H), 3.31-3.27(m, 1H), 2.55(t, J=6.2 Hz, 2H), 2.48(t, J=6.2 Hz, 2H), 1.98(s, 3H)

The resulting compound 34 can be derived to the oxazoline body by using existing oxazolination methods.

### «Example 5»

In this Example, compound 12 was prepared by a different step from that in Example 1.

### (Preparation of compound 36)

THF solution of 1.0 M BH₃ (32.6 mL, 32.6 mmol) and a dichloromethane solution of 1.0Mn-Bu₂BOTf (6.52 mL, 6.52 mmol) were added to compound 35 (4.07 g, 4.35 mmol) sequentially, and the mixture was stirred at 0°C for 1 hour under argon atmosphere. Triethylamine (12 mL) was added to the reaction solution to stop the reaction, and the reaction solution was concentrated under reduced pressure. The residue was purified by silica gel column chromatography (n-hexane:ethyl acetate = 3:2→1:1) to obtain white solid compound 36 (4.08 g, 88 %).

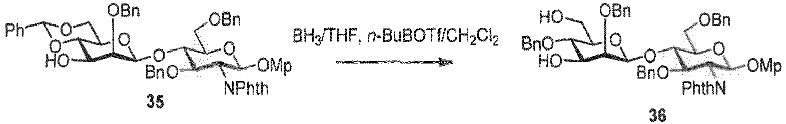

### (Preparation of compound 37)

Compound 36 (3.57 g, 3.81 mmol) was dissolved in methanol (48 mL), ethylenediamine (12 mL) was added, and the mixture was heated to reflux for 17 hours. The reaction solution was concentrated and azeotroped three times with DMF. Pyridine (30 mL) and acetic anhydride (15 mL) were added to the residue sequentially, and the mixture was stirred at room temperature for 18 hours. Methanol (10 mL) was added to the reaction solution to stop the reaction, and the mixture was concentrated under reduced pressure. Water was added, and the reaction solution was extracted twice with ethyl acetate. The organic phase was washed with 1M hydrochloric acid, saturated sodium hydrogen carbonate aqueous solution, and saturated saline in this order, then anhydrous magnesium sulfate was added for desiccation. After filtration of the desiccant, the solvent was removed under reduced pressure. The residue was purified by silica gel column chromatography (n-hexane:ethyl acetate=1:1) to obtain white solid compound 37 (3.45 g, 97 % in 2 steps).

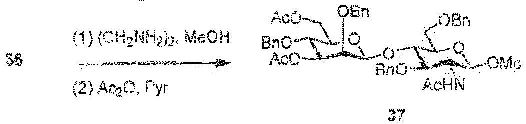

### (Preparation of compound 38)

Compound 37 (3.44 g, 3.68 mmol) was dissolved in methanol (60 mL), a catalytic amount of 28% sodium methoxide sodium solution was added, and the mixture was stirred at room temperature for 24 hours. Amberlite IR-120 (H⁺ form) was added to the reaction solution to neutralize it, and then the resin was separated by filtration. The filtrate was concentrated under reduced pressure, and the residue was purified by silica gel column chromatography (n-hexane:ethyl acetate = 2:3) to obtain white solid compound 38 (3.02 g, 96%).

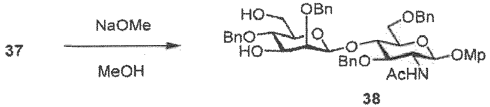

### (Preparation of compound 39)

Compound 38 (3.00 g, 3.52 mmol) and compound 3 (7.41 g, 14.5 mmol) were dissolved in DMF (15 mL), sodium hydride (323 mg, 1.84 mmol) was added under argon atmosphere, the mixture was stirred at 0°C for 1 hour, and then stirred at room temperature for 20 hours. 5% citric acid aqueous solution (9.0 mL) was added to the reaction solution to stop the reaction, then water was added, and the reaction solution was extracted twice with ethyl acetate. The organic phase was washed with saturated sodium hydrogen carbonate aqueous solution and saturated saline in this order, then anhydrous magnesium sulfate was added for desiccation. After filtration of the desiccant, the solvent was removed under reduced pressure. The residue was purified by silica gel column chromatography (ethyl acetate) to obtain colorless oily compound 39 (3.98 g, 74 %).

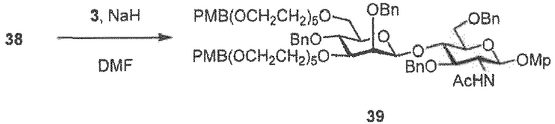

### (Preparation of compound 40)

Compound 39 (3.98 g, 2.60 mmol) was dissolved in dichloromethane (60 mL), a mixture of dichloromethane (54 mL) -TFA (5.7 mL) -water (0.3mL) was added, and a mixture was stirred at room temperature for 2.5 hours. Saturated sodium hydrogen carbonate aqueous solution (240 mL) was added to the reaction solution, the mixture was stirred for 1 hour to stop the reaction, then water was added, and the reaction solution was extracted twice with ethyl acetate. The organic phase was washed with saturated sodium hydrogen carbonate aqueous solution and saturated saline in this order, then anhydrous sodium sulfate was added for desiccation. After filtration of the desiccant, the solvent was removed under reduced pressure. The resulting residue (5.27 g) was dissolved in methanol (40 mL), a catalyst amount of 28% sodium methoxide sodium solution was added, and the mixture was stirred at room temperature for 2 hours. Amberlite IR-120 (H⁺ form) was added to the reaction solution to neutralize it, and then the resin was separated by filtration. The filtrate was concentrated under reduced pressure, and the residue was purified by silica gel column chromatography (ethyl acetate- chloroform: methanol = 20:1) to obtain pale yellow oily compound 40 (3.26 g, 97% in 2 steps).

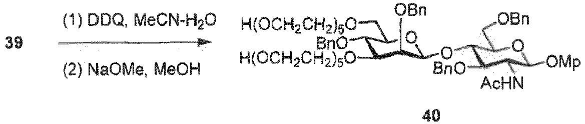

### (Preparation of compound 41)

Compound 40 (3.26 g, 2.53 mmol) was dissolved in dichloromethane (30 mL), triethylamine (4.24 mL, 30.3 mmol)) and tosyl chloride (2.89 g, 15.2 mmol) were added sequentially, and the mixture was stirred at room temperature for 16 hours. The reaction solution was diluted with chloroform and extracted with saturated sodium hydrogen carbonate solution. The organic phase was washed with saturated saline, and then dried with anhydrous sodium sulfate. After filtration of the desiccant, the solvent was removed under reduced pressure. The residue was purified by silica gel column chromatography (chloroform:methanol = 50:1) to obtain yellow oily compound 41 (3.48 g, 86 %).

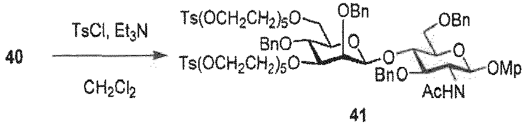

### (Preparation of compound 42)

Compound 41 (3.45 g, 2.16 mmol) was dissolved in 1,4-dioxane (50 mL), the mixture was added to a suspension of Pd/C (2.86 g) in 1,4-dioxane (10 mL), then water (10 mL) was added, and the mixture was stirred for 22 hours at room temperature under hydrogen atmosphere. The solid matter was separated by filtration and washed with 50% 1, 4-dioxane aqueous solution, and then the filtrate and washing solution were combined and concentrated under reduced pressure. The resulting residue was purified by silica gel column chromatography (chloroform:methanol:water = 9:1:0.06) to obtain colorless oily compound 42 (2.51 g, 94 %).

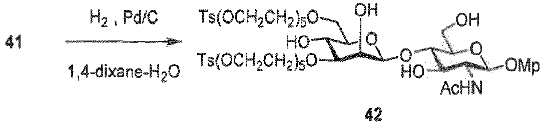

### (Preparation of compound 43)

Compound 42 (2.41 g, 1.95 mmol) was dissolved in DMF (25 mL), sodium azide (1.27 g, 19.5 mmol) was added, and the mixture was stirred at 50°C for 20 hours. The reaction solution was concentrated under reduced pressure. The resulting residue was purified by silica gel column chromatography (chloroform:methanol:water = 9:1:0.06) to obtain colorless oily compound 43 (1.76 g, 92%).

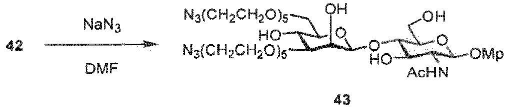

### (Preparation of compound 44)

Compound 43 (1.75 g, 1.79 mmol) was dissolved in pyridine (8.0 mL), acetic anhydride (4.0 mL) was added, and the mixture was stirred at room temperature for 26 hours. Methanol (8 mL) was added to dissolve the excess reagent, and the reaction solution was concentrated under reduced pressure. Water was added to the residue, and the reaction solution was extracted twice with ethyl acetate. The organic phase was washed with 1M hydrochloric acid, saturated sodium hydrogen carbonate aqueous solution, and saturated saline in this order, then anhydrous magnesium sulfate was added for desiccation. After filtration of the desiccant, the solvent was removed under reduced pressure. The residue was purified by silica gel column chromatography (chloroform:methanol:water = 20:1) to obtain colorless oily compound 44 (2.07, quant.).

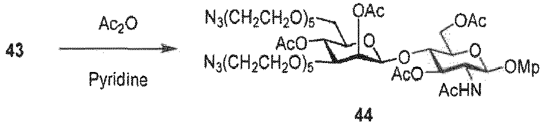

### (Preparation of compound 45)

Compound 44 (1.50 g, 1.31 mmol) was dissolved in 25% acetonitrile solution (28 mL), CAN (2.15 g, 3.92 mmol) was added, and the mixture was stirred at 0°C for 2.5 hours. The reaction solution was diluted with chloroform and divided twice with water. The organic phase was washed with saturated sodium hydrogen carbonate aqueous solution and saturated saline in this order, and then dried with anhydrous magnesium sulfate. After filtration of the desiccant, the solvent was removed under reduced pressure. The residue was purified by silica gel column chromatography (chloroform:methanol = 40:1→20:1) to obtain orange oily compound 45 (900 mg, 66%).

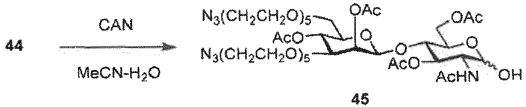

### (Preparation of compound 46)

Compound 45 (900 mg, 864 µmol) was dissolved in dichloromethane (18 mL), triethylamine (724 µL, 5.18 mmol) and DMC (438 mg, 2.59 mmol) were added sequentially, and the mixture was stirred at room temperature for 1.5 hours. The reaction solution was diluted with chloroform, and saturated sodium hydrogen carbonate solution was added to divide five times. The organic phase was washed with saturated saline and dried with anhydrous magnesium sulfate. After filtration of desiccant, the solvent was removed under reduced pressure. The residue was purified by silica gel column chromatography (chloroform:methanol = 60:1 (with 1% triethylamine)) to obtain orange oily compound 46 (660 mg, 75%).

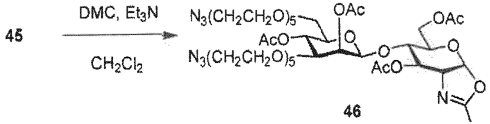

### (Preparation of compound 12)

Compound 46 (16.1 mg, 27.3 µmol) was dissolved in methanol (3.0 mL), a catalyst amount of 28% sodium methoxide sodium solution was added, and the mixture was stirred at room temperature for 18 hours. The reaction solution was concentrated under reduced pressure to obtain pale yellow oily compound 12.

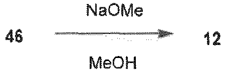

### «Example 6»

In this Example, the drug was introduced into the azide group of compound 12.

### (Preparation of compound 49)

Triethylamine (16.1 µL, 116 µmol) and 100 mM DMSO solution of compound 48 (462 µL, 46.2 µmol) were added to a 100 mM DMSO solution of compound 47 (358 µL, 38.5 µmol) sequentially, and the mixture was stirred at room temperature for 4 hours. The reaction solution was purified by silica gel column chromatography (chloroform:methanol = 15:1), and the resulting crude product (605 mg) was purified again with LH-20 (methanol). Water was added to the obtained crude product (450 mg), and the mixture was extracted twice with ethyl acetate. The organic phase was washed with water again and dried with magnesium sulfate. After filtration of desiccant, the solvent was removed under reduced pressure to obtain brown solid compound 49 (28.3 mg, 68%).

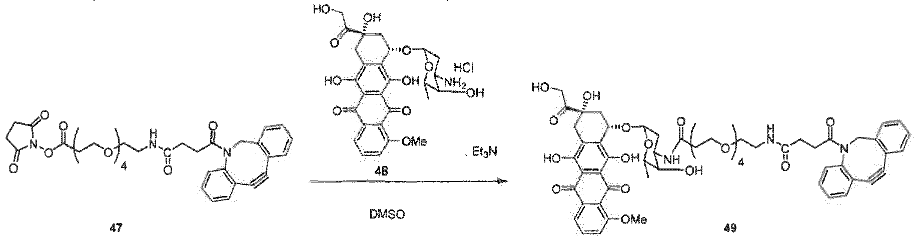

### (Preparation of compound 50)

A 10 mM methanol solution of compound 11 (242 µL, 2.42 µmol) was added to a 10 mM chloroform-methanol solution of compound 49 (1.94 mL, 19.4 µmol), and the mixture was stirred at room temperature for 3 hours. The reaction solution was concentrated under reduced pressure, and the residue was purified by silica gel column chromatography (chloroform:methanol = 85:15→chloroform:methanol:water = 8:2:0.15) to obtain brown solid compound 50 (4.2 mg, 57 %).

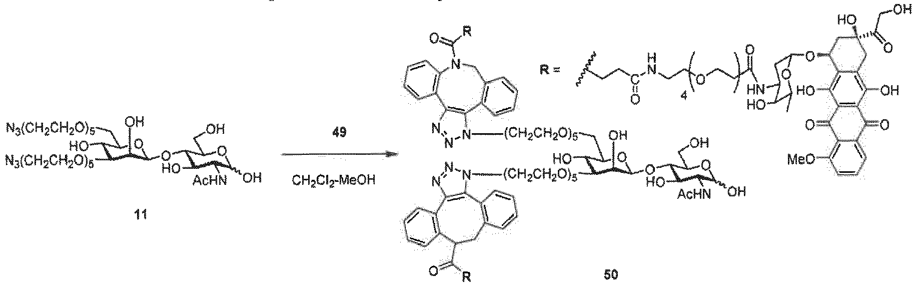

### (Preparation of compound 51)

Compound 50 (3.1 mg, 1.02 µmol) was dissolved in a mixture of deuterated chloroform (0.2 mL) and deuterated methanol (0.2 mL), 2-chloro-1,3-dimethylimidazolinium chloride (5.2 mg, 30.7 µmol) and triethylamine (10.7 µL, 76.7 µmol) were added sequentially, and the mixture was stirred at 0°C overnight. MALDI-TOFMS of the reaction solution confirmed the preparation of compound 51. MALDI-TOFMS.:Calcd. forC₁₄₈H₁₈₇N₁₃O₅₄Na, *m*/*z*[M+Na]⁺:3033.2, Found3031.6.

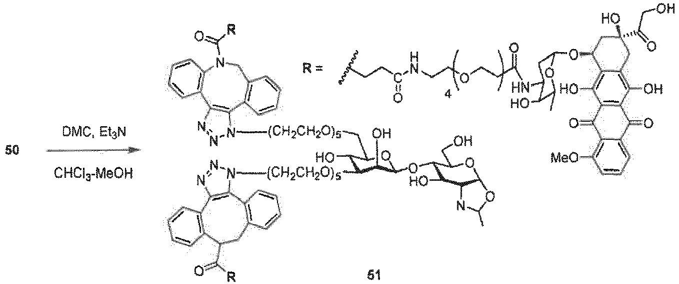

### «Example 7»

In this Example, a sugar compound having a PEG chain with a substituted tetrazine group at the end was prepared.

### (Preparation of compound 53)

THF solution of 1.0MBH₃ (17.7 mL, 17.7 mmol) and a dichloroethane solution of 1.0Mn-Bu₂BOTf (3.35 mL, 3.35 mmol) were added to compound 52 (2.46 g, 2.36 mmol) sequentially, and the mixture was stirred at 0°C for 1 hour under argon atmosphere. Triethylamine (5.0 mL) was added to the reaction solution to stop the reaction, and the mixture was concentrated under reduced pressure. The residue was purified by silica gel column chromatography (toluene:ethyl acetate = 4:1) to obtain white powdery compound 53 (2.32 g, 99 %).

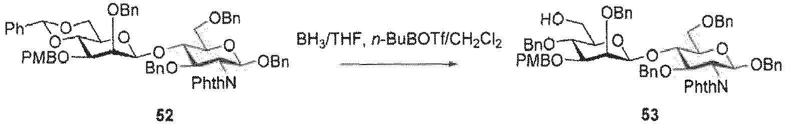

### (Preparation of compound 54)

Compound 53 (2.22 g, 2.13 mmol) was dissolved in dichloromethane (20 mL), triethylamine (1.78 mL, 12.8 mmol) and mesyl chloride (495 µL, 6.39 mmol) were added sequentially, and the mixture was stirred at 60 °C for 24 hours. Water was added to the reaction solution, and the mixture was extracted twice with chloroform. The organic phase was washed with saturated sodium hydrogen carbonate solution and saturated saline in this order, and then dried with anhydrous magnesium sulfate. After filtration of the desiccant, the solvent was removed under reduced pressure. The residue was purified by silica gel column chromatography (toluene: ethyl acetate = 7:1) to obtain white powdery compound 54 (1.27 g, 62 %).

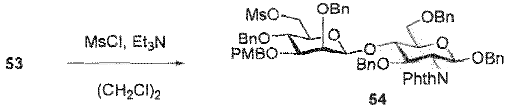

### (Preparation of compound 55)

Compound 54 (1.27 g, 1.13 mmol) was dissolved in DMF (13 mL), sodium azide (368 mg, 5.67 mmol) was added, and the mixture was stirred at 80°C for 15 hours. Water was added to the reaction solution, and the mixture was extracted twice with ethyl acetate. The organic phase was washed with saturated sodium hydrogen carbonate aqueous solution and saturated saline in this order, and then dried with anhydrous magnesium sulfate. After filtration of the desiccant, the solvent was removed under reduced pressure. The residue was purified by silica gel column chromatography (toluene: ethyl acetate = 8:1) to obtain white powdery compound 55 (1.11 g, quant.).

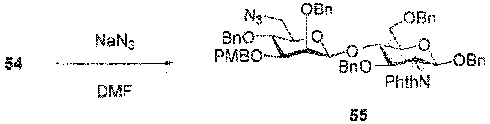

### (Preparation of compound 56)

Compound 55 (1.16 g, 1.09 mmol) was dissolved in a mixture of methanol (12 mL)-THF (3.0 mL), ethylenediamine (3.0 mL) was added, and the mixture was heated to reflux for 18 hours. The reaction solution was concentrated under reduced pressure, and azeotroped three times with DMF. Pyridine (4.5 mL) and acetic anhydride (3.0 mL) were added to the residue sequentially, and the mixture was stirred at room temperature for 18 hours. Methanol (4.5 mL) was added to the reaction solution to stop the reaction, and the mixture was concentrated under reduced pressure. Water was added to the residue, and the mixture was extracted twice with ethyl acetate. The organic phase was washed with 1M hydrochloric acid, saturated sodium hydrogen carbonate aqueous solution, and saturated saline in this order, then anhydrous magnesium sulfate was added for desiccation. After filtration of the desiccant, the solvent was removed under reduced pressure. The residue was purified by silica gel column chromatography (toluene: ethyl acetate = 3:1) to obtain white solid compound 56 (1.08 g, 98 % in 2 steps).

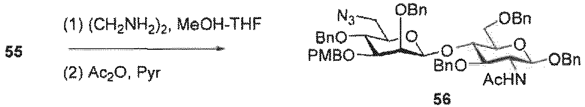

### (Preparation of compound 57)

Compound 56 (195 mg, 199 µmol) was dissolved in 1,4-dioxane (10 mL), the mixture was added to a suspension of Pd/C (128 mg) in 1,4-dioxane (2.0 mL), then water (10mL) and 1M hydrochloric acid () were added sequentially, and the mixture was stirred at room temperature for 21 hours under a hydrogen atmosphere. The solid matter was separated by filtration, washed with 50% 1, 4-dioxane aqueous solution, and the filtrate and washing solution were combined and concentrated under reduced pressure to obtain compound 57 (79.0 mg).

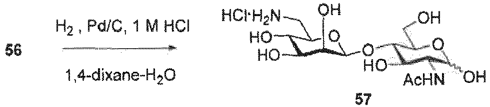

### (Preparation of compound 59)

tert-Butyl 12-hydroxy-4,7,10-trioxadodecanoate (compound 58) (204 mg, 0.73 mmol) was dissolved in dichloromethane (7 mL), triethylamine (305 µL, 2.19 mmol) and tosyl chloride (419 mg, 2.19 mmmol) were added, and the mixture was stirred at room temperature for 18 hours. The reaction solution was diluted with chloroform, washed with 5% citric acid solution and saturated saline, and dried with anhydrous magnesium sulfate. After filtration of the desiccant, the solvent was removed under reduced pressure. The resulting residue was dissolved in DMF (7 mL), sodium azide (233 mg, 3.60 mmmol) was added, and the mixture was stirred at 65°C for 18 hours. The reaction solution was diluted with chloroform, washed with water and saturated saline, and dried with anhydrous magnesium. After filtration of the desiccant, the solvent was removed under reduced pressure. The residue was purified by silica gel chromatography (n-hexane:ethyl acetate = 3:1) to obtain colorless oily compound 59 (209 mg, 96%).

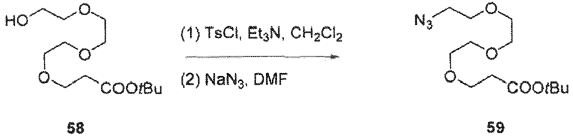

### (Preparation of compound 60)

Compound 59 (185 mg, 0.61 mmol) was dissolved in ethyl acetate (8 mL), palladium-activated carbon ethylenediamine complex (167 mg) was added, and the mixture was stirred at room temperature for 1 hour under hydrogen atmosphere. The reaction solution was filtered through celite, and the solvent was removed under reduced pressure to obtain compound 60 quantitatively.

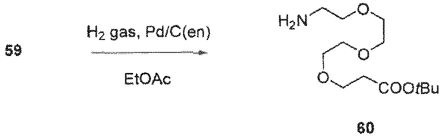

### (Preparation of compound 62)

Compound 61 (84.0 mg, 0.39 mmol) was dissolved in DMF (4 mL), DIEA (82 µL, 0.47 mmol) and PyBOP (243 mg, 0.47 mmol) were added sequentially, and the mixture was stirred at room temperature for 10 minutes. Then, compound 60 (107 mg, 0.39 mmol) was added, and the mixture was stirred at room temperature for 18 hours. The reaction solution was diluted with ethyl acetate, washed with saturated sodium carbonate aqueous solution, water, and saturated saline, and dried with anhydrous magnesium sulfate. After filtration of the desiccant, the solvent was removed under reduced pressure. The residue was purified by silica gel chromatography (n-hexane: ethyl acetate = 1:5) to obtain red solid compound 62 (132 mg, 71 %).

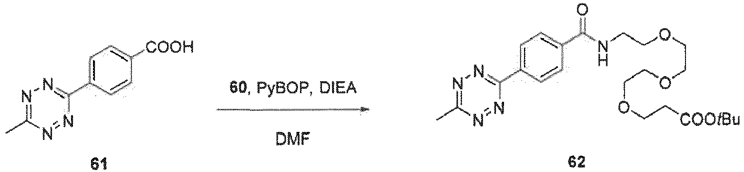

### (Preparation of compound 63)

Compound 62 (62.2 mg, 137 µmol) was dissolved in dichloromethane (2.0 mL), trifluoroacetic acid (0.2 mL) was added, and the mixture was stirred at room temperature for 4 hours. The reaction solution was concentrated under reduced pressure, toluene was added and azeotroped three times. The residue was purified by silica gel column chromatography (chloroform:methanol:water = 9:1:0.06) to obtain pink solid compound 63 (54.1 mg, 94 %).

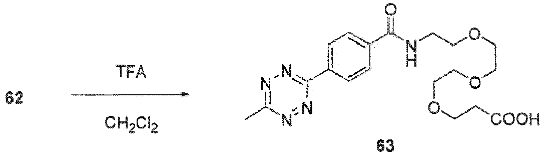

### (Preparation of compound 64)

Compound 63 (52.6 mg, 125 µmol) was dissolved in dichloromethane (3.8 mL), and 1-(3-dimethylaminopropyl)-3-ethylcarbodiimide hydrochloride (52.9 mg, 276 µmol) and HOSu (19.5 mg, 169 µmol) were added sequentially, and the mixture was stirred at room temperature for 16 hours. Water was added to the reaction solution, and the mixture was extracted twice with ethyl acetate. The organic phase was washed with 5 % citric acid aqueous solution, saturated sodium hydrogen carbonate aqueous solution, and saturated saline in this order, and then dried with magnesium sulfate. After filtration of the desiccant, the solvent was removed to obtain pink solid compound 64 (63.3mg, 86% (NMR yield)).

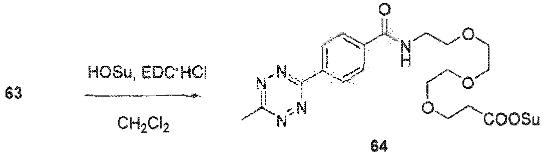

### (Preparation of compound 65)

Compound 57 (79.0 mg, 189 µmol) was dissolved in water (0.85 mL), triethylamine (13.3 µL, 94.5 µmol) and compound 64 (24.4 mg, 47.2 µmol) in acetonitrile solution (0.8 mL) were added sequentially, and the mixture was stirred at room temperature for 2 hours. Water (3 mL) was added to the reaction solution, the mixture was frozen, and then lyophilized. The residue was purified by silica gel column chromatography (chloroform:methanol:water = 8:2:0.15→7:3:0.3) to obtain pink solid compound 65 (28.7 mg, 78 %).

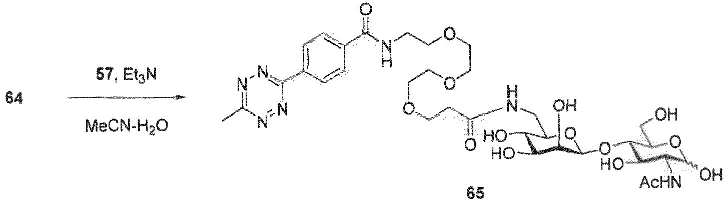

### (Preparation of compound 66)

Compound 65 (3.1 mg, 3.96 µmol) was dissolved in a mixture of trideutero acetonitrile (0.20 mL) and deuterium water (0.10 mL), 2-chloro-1,3-dimethylimidazolinium chloride (20.0 mg, 119 µmol) and triethylamine (41.5 µL, 297 µmol) were added sequentially, and the mixture was stirred at 0°C overnight. MALDI-TOF MS of the reaction solution confirmed the preparation of compound 66. MALDI-TOFMS.:Calcd. for C₃₃H₄₇N₇O₁₄Na *m*/*z*[M+Na]⁺:788.3, Found 788.7.

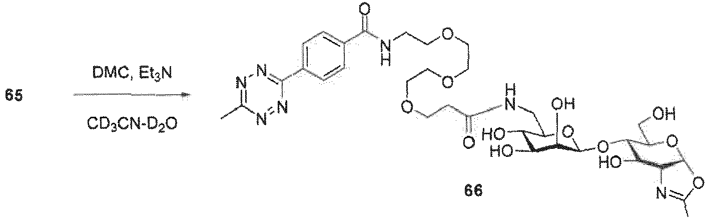

### «Example 8»

In this Example, a branched sugar compound having a PEG chain with an azide group and a substituted tetrazine group at the end was prepared.

### (Preparation of compound 67)

Compound 59 (557 mg, 1.83 mmol) was dissolved in dichloromethane (25 mL), trifluoroacetic acid (2.6 mL, 34 mmol) was added under ice-cold conditions, and the mixture was stirred at room temperature for 3 hours. Toluene was added to the reaction solution, the mixture was concentrated, and then the residue was purified by silica gel column chromatography (n-hexane: ethyl acetate = 1:2) to obtain compound 67 (375 mg, 83%).

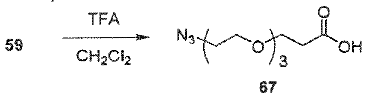

### (Preparation of compound 68)

H-Lys (Boc)-OtBu hydrochloride (122 mg, 0.36 mmol) and compound 67 (89 mg, 0.36 mmol) were dissolved in DMF (4mL), DIEA (138 µL, 0.79 mmol) and PyBOP (255 mg, 0.43 mmol) were added sequentially, and the mixture was stirred at room temperature for 18 hours, and stirred at room temperature for 18 hours. The reaction solution was diluted with ethyl acetate, washed with saturated sodium carbonate aqueous solution, water, and saturated saline, and dried with anhydrous magnesium sulfate. After filtration of the desiccant, the solvent was removed under reduced pressure. The residue was purified by silica gel chromatography (n-hexane: ethyl acetate = 1:3) to obtain white solid compound 68 172 mg, 90 %).

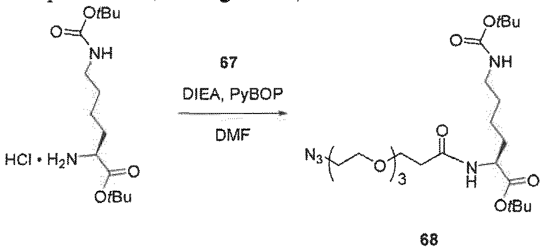

### (Preparation of compound 69)

Compound 68 (170 mg, 0.32 mmol) was dissolved in 4 M hydrogen chloride -1,4-dioxane solution (4 mL), and the mixture was stirred at room temperature for 4 hours. The reaction solution was concentrated under reduced pressure and lyophilized to obtain colorless oily compound 69 (128 mg, 98 %).

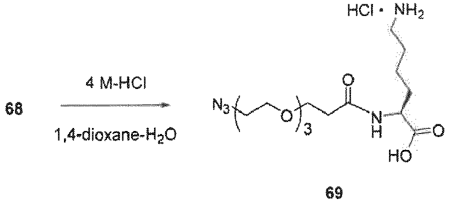

### (Preparation of compound 70)

Compound 69 (270 mg, 0.43 mmol) and compound 64 (182 mg, 0.44 mmol) were dissolved in DMF (5 mL), triethylamine (178 µL, 1.28 mmol) was added, and the mixture was stirred at room temperature for 3 hours. The reaction solution was removed under reduced pressure, and the residue was purified by silica gel chromatography (chloroform:methanol:water = 10:1:0.06) to obtain pink solid compound 70 (347 mg, 96 %).

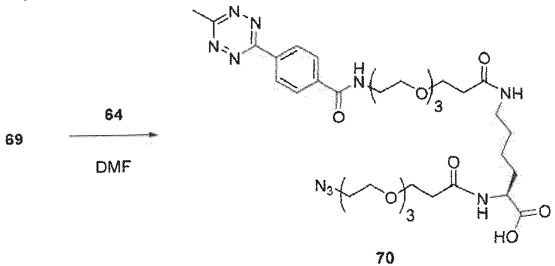

### (Preparation of compound 71)

Compound 70 (10.4 mg, 13.3 µmol) was dissolved in acetonitrile (0.5 mL), HOSu (2.2 mg, 19.1 µmol) and 1-(3-dimethylaminopropyl)-3-ethylcarbodiimide hydrochloride (3.8 mg, 19.8 µmol) were added sequentially, and the mixture was stirred at room temperature overnight. HOSu (1.1 mg, 9.6 µmol) and 1-(3-dimethylaminopropyl)-3-ethylcarbodiimide hydrochloride (1.9 mg, 9.9 µmol) were added to the reaction solution sequentially, and the mixture was stirred at room temperature for 5 hours. An aqueous solution (150 µL) of compound 57 (5.0 mg, 13.3 µmol) and triethylamine (3.7 µL, 26.6 µmol) were added to the reaction solution sequentially, and the mixture was stirred overnight at room temperature. After lyophilization of the reaction solution, the residue was purified by silica gel chromatography (chloroform:methanol = 80:20→70:30) to obtain pink solid compound 71 (3.1 mg, 20%).

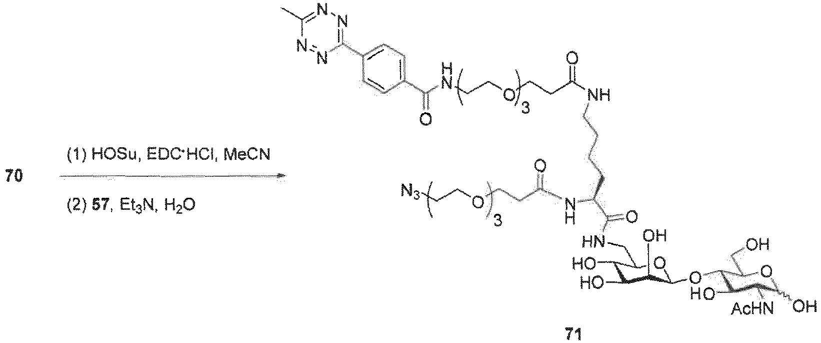

### (Preparation of compound 72)

Compound 71 (3.1 mg, 2.72 µmol) was dissolved in a mixture of trideutero acetonitrile (0.10 mL) and deuterium water (0.10 mL), 2-chloro-1,3-dimethylimidazolinium chloride (13.8 mg, 81.5 µmol) and triethylamine (28.5 µL, 204 µmol) were added sequentially, and the mixture was stirred at 0°C overnight. MALDI-TOFMS of the reaction solution confirmed the preparation of compound 72. MALDI-TOF MS.:Calcd. for C₄₈H₇₄N₁₂O₁₉Na *m*/*z*[M+Na]⁺:1145.5, Found1146.3.

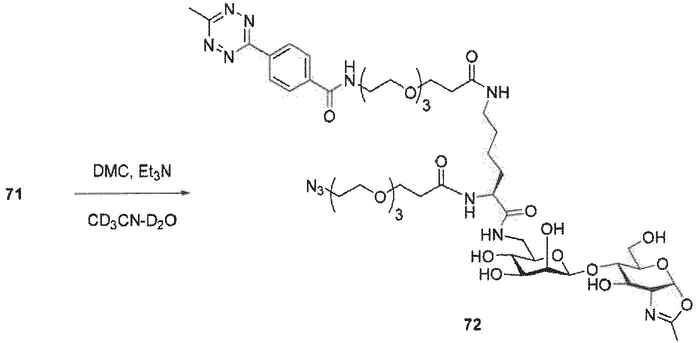

### «Example 9»

In this Example, a sugar compound having a PEG chain with an azide group at the end was prepared.

### (Preparation of compound 74)

Compound 73 (55.9 mg, 192 µmol) was dissolved in dichloromethane (2 mL), N-hydroxysuccinimide (39.0 mg, 339 µmol) and 1-ethyl-3-(3-dimethylaminopropyl) carbodiimide hydrochloride (64.7 mg, 338 µmol) were added sequentially, and the mixture was stirred at room temperature overnight. Water was added to the reaction solution, and the mixture was extracted with chloroform twice. The organic phase was washed with water and saturated saline in this order, then anhydrous magnesium sulfate was added for desiccation. After filtration of the desiccant, the solvent was removed under reduced pressure to obtain compound 74 (72.4 mg, 97 %).

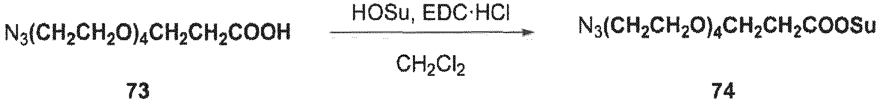

### (Preparation of compound 75)

Compound 43 (45.2 mg, 46.1 µmol) was dissolved in dioxane (1 mL) and 1,4-dioxane suspension (3 mL) of 10% palladium-activated carbon (29.47 mg) was added, and then water (1 mL) was added. The mixture was stirred overnight at room temperature under hydrogen atmosphere. The solid matter was separated by filtration, washed with a 50% 1,4-dioxane aqueous solution, and the filtrate and washing solution were combined and concentrated under reduced pressure. Compound 74 (72.4 mg, 186 µmol) in a 50% acetonitrile aqueous solution (1.6 mL) and triethylamine (54 µL, 390 µmol) was added to the resulting residue. After stirring at room temperature overnight, water (2.0 mL) was added to the reaction solution, and the mixture was lyophilized. The residue was purified by silica gel column chromatography (CHCl₃-MeOH-H₂O = 9:1:0.06). The purified product was added to pyridine (3 mL) and acetic anhydride (1.5 mL), and the mixture was stirred overnight at room temperature. Methanol (4mL) was added to the reaction solution to stop the reaction, and the mixture was concentrated under reduced pressure. After azeotropy with toluene (4 mL) was repeated five times, the residue was purified by silica gel chromatography (CHCl₃-MeOH-H₂O = 15:1:0.04) to obtain compound 75 (30.4 mg, 40% in 3 steps).

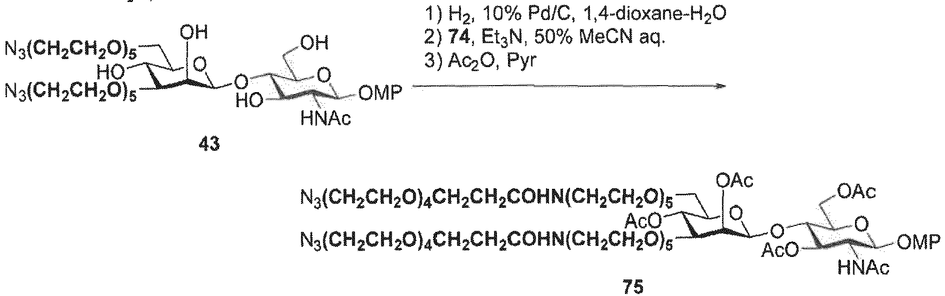

### (Preparation of compound 76)

Compound 75 (8.7 mg, 5.3 µmol) was dissolved in acetonitrile (2 mL), water (0.5 mL) and ammonium cerium (IV) nitrate (11.2 mg, 21 µmmol) were added at 0°C, and the mixture was stirred for 1.5 hours, followed by 2 hours at room temperature. Further, ammonium cerium (IV) nitrate (10.5 mg, 19 µmmol) was added to the reaction mixture, and the mixture was stirred at 0°C for 1.5 hours. Saturated sodium hydrogen carbonate aqueous solution was added to the reaction solution, and the mixture was extracted twice with chloroform. The organic phase was washed with saturated saline, then anhydrous magnesium sulfate was added for desiccation. After filtration of the desiccant, the solvent was removed under reduced pressure. The residue was purified by silica gel column chromatography (CHCl₃-MeOH-H₂O = 9:1:0.06) to obtain compound 76 (4.6 mg, 57%).

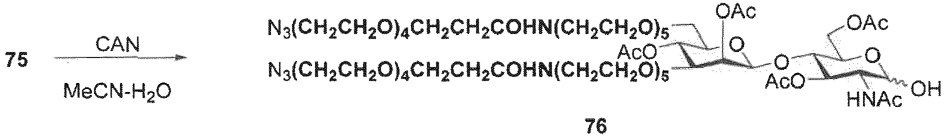

### (Preparation of compound 77)

Compound 76 (4.6 mg, 3.0 µmol) was dissolved in dichloromethane (1 mL), triethylamine (20 µL, 140 µmmol) and 2-chloro-1,3-dimethylimidazolinium chloride solution (0.2 M, 180 µL, 36 µmol) in dichloromethane were added, and the mixture was stirred at room temperature for 2 hours. Water was added to the reaction solution, and the mixture was extracted with chloroform twice. The organic phase was washed with saturated sodium hydrogen carbonate aqueous solution and saturated saline in this order, and then magnesium sulfate was added for the desiccation. After filtration of the desiccant, the solvent was removed under reduced pressure. The residue was purified by silica gel column chromatography (CHCl₃-MeOH-H₂O = 20:1:0.2) to obtain compound 77 (4.0 mg, 89%).

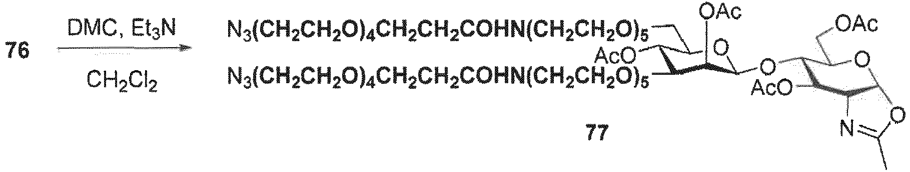

### (Preparation of compound 78)

Compound 77 (4.0 mg, 2.6 µmol) was dissolved in methanol (1 mL), sodium methoxide (0.5 mol/L methanol solution, 2 µL, 1 µmol) was added at 0°C, and then water (5 mL) was added, and the mixture was stirred for 1 hour, followed by 1 hour at room temperature. After the solvent was removed under reduced pressure, MALDI-TOFMS confirmed the presence of compound 78. 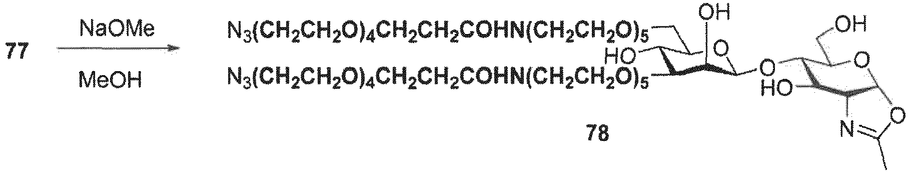 Compound 78 MALDI-TOF MS:[M+Na]⁺*m*/*z* 1372.4 (calcd.1372.7).

### «Example 10»

In this Example, a branched sugar compound having a PEG chain with an azide group at the end was prepared.

### (Preparation of compound 79)

L-lysine methyl ester dihydrochloride (108 mg, 462 µmol) and dichloromethane (1.6 mL) were added to compound 67 (305 mg, 1.23 mmol). To this solution, N,N-diisopropylethylamine (0.45 mL, 3.2 mmol) and 1-[bis(dimethylamino)methylene]-1h-benzotriazolium 3-oxide tetrafluoroborate (390 mg, 1.21 mmol) were added under ice-cold conditions, and the mixture was stirred at room temperature overnight. The reaction solution was concentrated, and then the residue was purified by silica gel column chromatography (chloroform:methanol = 60:1). The purified product was dissolved in chloroform, and the organic phase was washed with saturated sodium hydrogen carbonate aqueous solution, then anhydrous magnesium sulfate was added for desiccation. After filtration of the desiccant, the solvent was removed under reduced pressure to obtain compound 79 (283 mg, 98%).

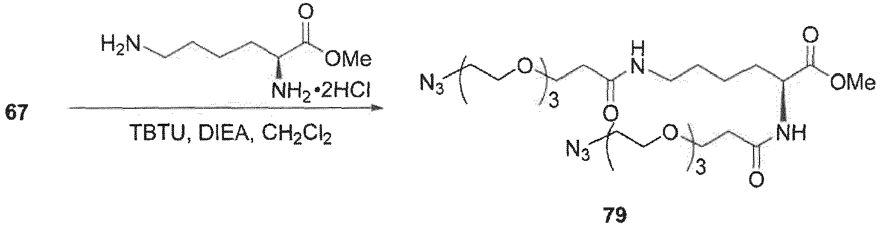

### (Preparation of compound 80)

Compound 79 (103 mg, 167 µmol) was dissolved in methanol (3.0 mL), 1 M sodium hydroxide aqueous solution (194 µL) was added under ice-cold conditions, and the mixture was stirred at room temperature for 5 hours. The reaction solution was extracted with water and washed with diethyl ether. 1M hydrochloric acid solution was added to the aqueous phase, pH was adjusted to 1, and then the mixture was extracted with ethyl acetate and chloroform, followed by desiccation by adding anhydrous magnesium sulfate. After filtration of the desiccant, the solvent was removed under reduced pressure to obtain compound 80 (71.7 mg, 72%).

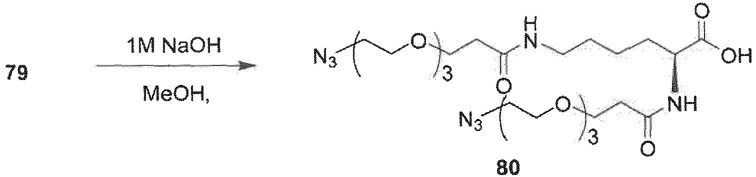

### (Preparation of compound 81)

Compound 80 (52 mg, 86 µmol) was dissolved in dichloromethane (0.86 mL), 1-(3-dimethylaminopropyl)-3-ethylcarbodiimide hydrochloride (36.5 mg, 190 µmol) and N-hydroxysuccinimide (13.4 mg, 116 µmol) were added, and then the mixture was stirred overnight at room temperature. Water was added to the reaction solution, and the mixture was extracted twice with chloroform. The organic phase was washed with saturated ammonium chloride aqueous solution and saturated saline in this order, and then dried with anhydrous magnesium sulfate. After filtration of the desiccant, the solvent was removed under reduced pressure to obtain compound 81 (52.3 mg, 87%).

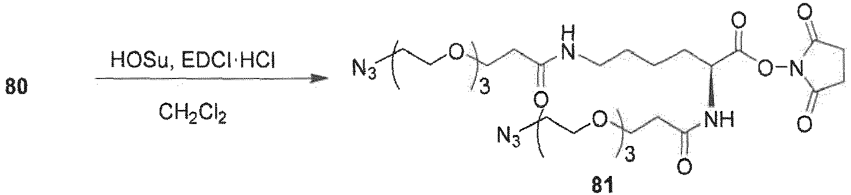

### (Preparation of compound 82)

Compound 56 (88.0 mg, 91.2 µmol) was dissolved in 1,4-dioxane (10.8 mL), palladium hydroxide (89 mg), water (2.64 mL), and 1M hydrochloric acid (136 µL) were added in this order, and the mixture was stirred overnight at room temperature under hydrogen atmosphere. Solid matter was separated by filtration, washed with 50% 1,4-dioxane solution, and the filtrate and washing solution were combined and concentrated under reduced pressure. The resulting residue and compound 81 (52.3 mg, 74.5 µmol) were dissolved in a 50% acetonitrile aqueous solution (0.8 mL), triethylamine (20 µL, 144 µmol) was added, and the mixture was stirred at room temperature for 5 hours. Water was added to the reaction solution, and after lyophilization, the residue was purified by silica gel column chromatography (chloroform:methanol:water = 7.5:2.5:0.3) to obtain compound 82 (22.7 mg, 38%).

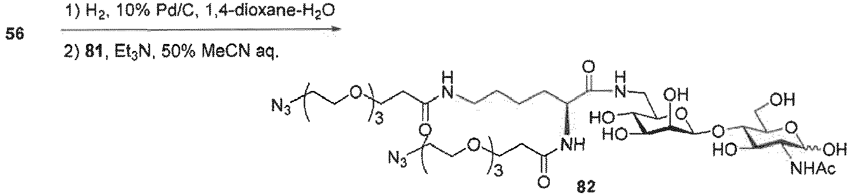

### (Preparation of compound 83)

Compound 82 (11.4 mg, 11.8 µmol) was dissolved in water (0.482 mL), 0.757M 2-chloro-1,3-dimethylimidazolinium chloride aqueous solution (0.482 mL, 365 µmol) and triethylamine (235 µL, 1.70 mmol) were added under ice-cold conditions, and then the mixture was stirred at room temperature for 18 hours. Water was added to the reaction solution, and after lyophilization, the residue was purified by gel filtration chromatography (0.05% triethylamine aqueous solution), and 0.1M sodium hydroxide solution (129 µL, 12.9 µmol) was added to the eluate containing compound HM9. The solution was lyophilized to obtain 22.0 mg of a mixture of compound 83, sodium hydroxide, and triethylamine. The structure of oxazoline was confirmed by 1H-NMR spectrum, and the amount of compound 83 was calculated from the integral value thereof to obtain compound HM9 (9.2 mg, 83%). 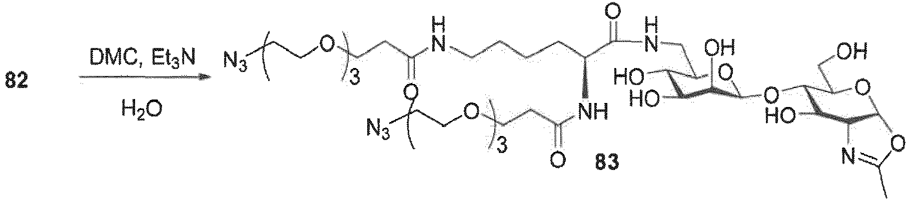 Compound 83 ¹H NMR (600MHz,D₂O) :δ=6.14 (d,J=6.14Hz,1H).

### «Example 11 »

In this Example, a sugar compound having three azide groups at the end was prepared.

### (Preparation of compound 84)

Compound 35 (797.4 mg, 852 µmol) was dissolved in methanol (6.80 mL), and ethylenediamine (1.70 mL) was added at room temperature. The reaction solution was stirred under heat reflux for 17 hours, diluted with dimethylformamide, and the solvent was removed under reduced pressure. The reaction solution was azeotroped with dimethylformamide three times, and the residue was dried in vacuo to obtain the crude product. The crude product was dissolved in methanol (6.82 mL) and purified water (1.70 mL), and then sodium hydrogen carbonate (218.3mg, 2.60mmol), copper (ii) sulfate pentahydrate (45.2 mg, 181 µmol), and imidazole-1-sulfonyl azide hydrochloride (271.3 mg, 1.29 mmol), and dichloromethane (8.52 mL) were added sequentially at room temperature. The reaction solution was stirred at room temperature for 1.5 hours, and copper (ii) sulfate pentahydrate (44.4 mg, 178 µmol) was added. The reaction solution was stirred at room temperature for 18 hours, and sodium hydrogen carbonate (152.3 mg,1.81 mmol) and imidazole-1-sulfonyl azide hydrochloride (359.3 mg, 1.71 mmol) were added. The reaction solution was stirred at room temperature for 3 hours, the reaction solution was diluted with chloroform, 1M hydrochloric acid aqueous solution was added, and the mixture was extracted with chloroform three times. The organic phase was washed with saturated sodium hydrogen carbonate aqueous solution and extracted with chloroform three times. The organic phase was dried with anhydrous magnesium sulfate, the residue was separated by filtration, and the solvent was removed under reduced pressure. The residue was purified by silica gel column chromatography (toluene:ethyl acetate = 7:1) to obtain colorless solid compound 84 (641.8 mg, 91%). 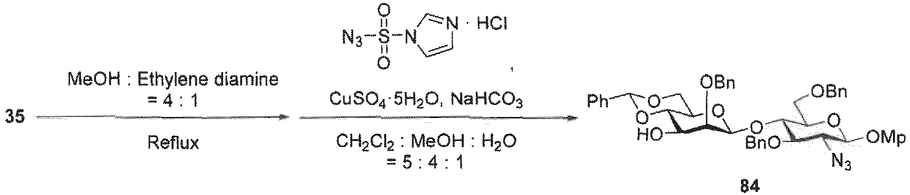 Compound 84 ¹H NMR (600 MHz, CDCl₃) δ:7.46 (dd, J=7.2, 1.7 Hz, 2H), 7.42-7.27 (m,18H), 7.06-7.02 (m, 2H), 6.85-6.81 (m, 2H), 5.45 (s,1H) ,5.08 (d,J=11.0 Hz,1H) ,4.93 (d, J=11.0 Hz,1H) ,4.72-4.67 (m, 3H), 4.66 (d, J=11.7 Hz,1H), 4.61 (s,1H), 4.47 (d, J=11.7 Hz, 1H) , 4.09 (dd, J=10.3, 4.8 Hz, 1H), 4.06 (t, J=9.3 Hz, 1H), 3.78 (s, 3H), 3.76-3.71 (m, 2H), 3.70-3.65 (m, 3H), 3.57 (td, J=9.3, 3.2 Hz, 1H), 3.49 (t, J=10.3 Hz, 1H), 3.47-3.44 (m, 1H), 3.41 (t, J=9.6 Hz, 1H), 3.10 (td, J=9.6, 4.8 Hz, 1H), 2.33 (d, J=8.2 Hz, 1H).
MALDI-TOFMS (AXIMA-TOF²) *m*/*z*[M+Na]⁺calcd. for C₄₇H₄₉N₃O₁₁Na, 854.3, found 854.2.

### (Preparation of compound 85)

Compound 84 (460.1 mg, 553.4 µmol) was dissolved in dichloromethane (4.61 mL), purified water (461 µL) was added at room temperature, and trifluoroacetic acid (461 µL) was added under ice-cold conditions. The reaction solution was stirred at room temperature for 20 hours, diluted with chloroform, saturated sodium carbonate aqueous solution was added, and the mixture was extracted with chloroform three times. The organic phase was dried with anhydrous sodium sulfate, the residue was separated by filtration, and the solvent was removed under reduced pressure. The residue was purified by silica gel chromatography (toluene:ethyl acetate = 1:2) to afford colorless solid compound 85 (379.8 mg, 92%). 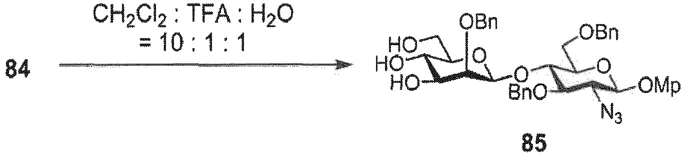 Compound 85 ¹H NMR (600 MHz,CDCl₃) δ:7.38-7.27 (m, 15H), 7.06-7.01 (m, 2H), 6.85-6.80 (m, 2H) , 5.06 (d, J=11.0 Hz, 1H), 4.97 (d, J=11.0 Hz, 1H) , 4.71 (d, J=8.9 Hz, 2H) , 4.69 (d, J=12.4 Hz, 1H) , 4.57 (d, J=11.0 Hz, 1H) , 4.52 (s, 1H), 4.49 (d, J=11.7 Hz, 1H), 4.00 (t, J=9.6 Hz, 1H), 3.78 (s, 3H), 3.76 (dd, J=11.0, 2.1 Hz, 1H), 3.71-3.63 (m, 3H), 3.61 (d, J=3.4 Hz, 1H), 3.55-3.49 (m, 2H), 3.43-3.36 (m, 2H) , 3.22 (td, J=8.9, 2.7 Hz, 1H), 3.05-3.02 (m, 1H), 2.37 (s, 1H), 2.35 (s, 1H), 2.25 (d, J=10.3 Hz, 1H).
MALDI-TOF MS (AXIMA-TOF²)
m/z [M+Na]⁺calcd. for C₄₀H₄₅N₃O₁₁Na, 766.3, found765.5.

### (Preparation of compound 86)

Compound 85 (379.8 mg, 511 µmol) and compound 3 (1.576 g, 3.07 mmol) were azeotroped three times with toluene, after vacuum drying, the dried matter was dissolved in dimethylformamide (5.10 mL), and sodium hydride (370.1mg, 8.49mmol, 45% oil complex) was added. The reaction solution was stirred at room temperature for 20.5 hours, diluted with ethyl acetate, 1M hydrochloric acid solution was added, and the mixture was extracted with ethyl acetate three times. The organic phase was dried with anhydrous sodium sulfate, the residue was separated by filtration, and the solvent was removed under reduced pressure. The residue was crudely purified by silica gel column chromatography (chloroform:methanol = 50:1). The resulting mixture was purified by gel filtration chromatography (methanol) to obtain colorless oily compound 86 (775.8 mg, 86%). 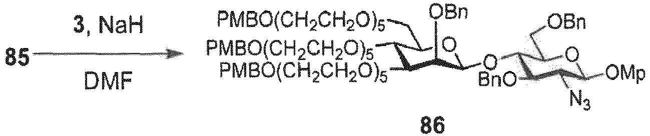 Compound 86 ¹H NMR (600 MHz,CDCl₃) δ:7.40-7.20 (m, 21H), 7.04-7.01 (m, 2H), 6.88-6.84 (m, 6H), 6.82-6.79 (m, 2H), 5.15 (d, J=11.0 Hz, 1H), 4.84 (d, J=11.7 Hz, 1H), 4.76 (d, J=12.4 Hz, 1H), 4.67 (d, J=8.2 Hz, 1H), 4.67 (d, J=11.0 Hz, 1H), 4.60 (d, J=11.7 Hz, 1H), 4.50-4.47 (m, 7H) , 4.45 (d, J=11.0 Hz, 1H), 3.97 (t, J=9.3 Hz, 1H), 3.94-3.90 (m, 1H), 3.81-3.76 (m, 10H), 3.73-3.57 (m, 56H) , 3.56-3.52 (m, 4H) , 3.52-3.49 (m, 4H) , 3.48-3.42 (m, 3H), 3.42-3.39 (m, 3H), 3.22 (ddd, J=9.6, 6.2, 1.4 Hz, 1H), 3.13 (dd, J=9.6, 2.7 Hz, 1H).
MALDI-TOF MS (AXIMA-TOF²)
m/z [M+Na]⁺calcd. for C₉₄H₁₂₉N₃O₂₉Na 1786.9, found1786.3.

### (Preparation of compound 88)

Zinc powder was suspended in water, the suspension was sonicated for 30 minutes, and stirred with 2% copper sulfate solution. The precipitate was separated by filtration and air-dried to obtain Zn-Cu complex. Compound 86 (635.0 mg, 359.8 µmol) was dissolved in tetrahydrofuran (18.0 mL) and acetic acid (18.0 mL), and Zn-Cu complex (1.19 g) was added. The reaction solution was stirred at room temperature for 2 hours, diluted with toluene, the solid was separated by filtration, the solvent was removed under reduced pressure, and the residue was dried in vacuo. This residue was dissolved in acetic anhydride (4.0 mL) and pyridine (4.0 mL). The reaction solution was stirred at room temperature for 18 hours, then diluted with toluene, and the solvent was removed under reduced pressure. The residue was azeotroped with toluene three times and dissolved in chloroform. Saturated sodium hydrogen carbonate aqueous solution was added, and the mixture was extracted with chloroform three times. The organic phase was dried with anhydrous sodium sulfate, the dried matter was separated by filtration, and the solvent was removed under reduced pressure. The residue was purified by silica gel column chromatography (chloroform:methanol = 50:1) to obtain crude product 87.

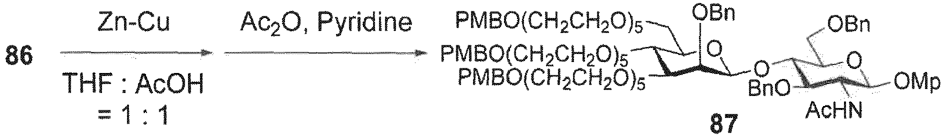

The crude product 87 was dissolved in dichloromethane (5.5 mL), 1,3-dimethoxybenzene (107.5 µL, 832 µmol) was added at room temperature, and trifluoromethanesulfonic acid (36.7 µL, 416 µmol) was added under ice-cold conditions. The reaction solution was stirred at room temperature for 1 hour, the mixture was diluted with chloroform, saturated sodium hydrogen carbonate aqueous solution was added, and the mixture was extracted with chloroform three times. The organic phase was dried with anhydrous sodium sulfate, the dried matter was separated by filtration, and the solvent was removed under reduced pressure. The residue was dried in vacuo to obtain the crude product. The crude product was dissolved in dichloromethane (2.77 mL) and methanol (2.77 mL), and 28% sodium methoxide methanol solution (50 µL) was added at room temperature. The reaction solution was stirred at room temperature for 6 hours, diluted with chloroform, the mixture was neutralized with AmberliteIR-120 (H⁺form), and the resin was separated by filtration. The filtrate was concentrated under reduced pressure, and the residue was purified by silica gel column chromatography (chloroform:methanol = 20:1) to obtain colorless oily compound 88 (322.6 mg, 63% in 4 steps). 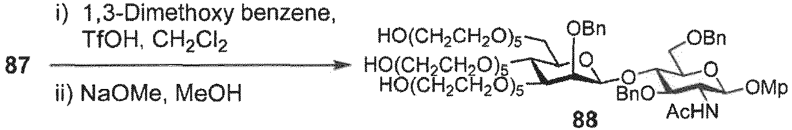 Compound 88 ¹H NMR (600 MHz,CDCl₃) δ:7.38 (d, J=6.9 Hz, 2H), 7.33-7.21 (m, 13H), 6.97 (d, J=8.9 Hz, 2H) , 6.80 (d, J=8.9 Hz, 2H) , 6.50 (d, J=8.2 Hz, 1H), 5.34 (d, J=5.5 Hz, 1H), 4.86 (d, J=11.7 Hz, 1H), 4.78 (s, 1H), 4.76 (s, 1H), 4.71 (d, J=11.7 Hz, 1H) , 4.50 (s, 1H), 4.46 (d, J=11.7 Hz, 1H), 4.41 (d, J=11.7 Hz, 1H), 4.17 (t, J=5.8 Hz, 1H), 4.07 (dd, J=14.1, 5.8 Hz, 1H), 3.98-3.93 (m, 2H), 3.91-3.84 (m, 2H), 3.83 (d, J=2.7 Hz, 1H) , 3.76 (s, 4H) , 3.74-3.47 (m, 59H) , 3.30-3.24 (m, 2H) , 2.88 (t, J=6.2 Hz, 2H) , 2.80 (t, J=6.2 Hz, 1H), 1.78 (brs, 6H) .
MALDI-TOFMS (AXIMA-TOF²) m/z[M+Na]⁺calcd for C₇₂H₁₀₉NO₂₇Na 1442.7, found1443.3.

### (Preparation of compound 89)

Compound 88 (322.6 mg, 227 µmol) was dissolved in dichloromethane (4.54 mL), triethylamine (286 µL, 2.05 mmol) and p-toluenesulfonyl chloride (358.4 mg, 2.10 mmol) were added sequentially at room temperature. The reaction solution was stirred at room temperature for 1 hour, and then 4-dimethylaminopyridine (8.4 mg, 68.8 µmol) was added. The reaction solution was stirred at room temperature for 18 hours, diluted with chloroform, saturated sodium hydrogen carbonate aqueous solution was added, and the mixture was extracted with chloroform three times. The organic phase was dried with anhydrous sodium sulfate, the dried matter was separated by filtration, and the solvent was removed under reduced pressure. The residue was purified by silica gel column chromatography (chloroform:methanol = 50:1) to obtain colorless oily compound 89 (403.4 mg, 94 %). 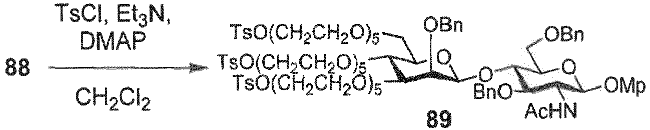 Compound 89 ¹H NMR (600 MHz, CDCl₃) δ:7.82-7.76 (m, 6H), 7.40-7.19 (m, 21H), 6.97 (d, J=8.9 Hz, 2H) , 6.79 (d, J=9.6 Hz, 2H) , 6.39 (d, J=8.9 Hz, 1H), 5.33 (d, J=5.5 Hz, 1H), 4.86 (d, J=11.7 Hz, 1H), 4.79-4.75 (m, 2H), 4.71 (d, J=11.7 Hz, 1H), 4.49 (s, 1H), 4.46 (d, J=11.7 Hz, 1H), 4.40 (d, J=12.4 Hz, 1H), 4.17-4.11 (m, 8H), 4.07 (dd, J=13.4, 5.8 Hz, 1H), 3.97-3.92 (m, 2H), 3.90-3.83 (m, 2H), 3.82 (d, J=2.7 Hz, 1H), 3.76 (s, 3H), 3.74-3.46 (m, 65H) , 3.29-3.22 (m, 2H), 1.80 (d, J=6.2 Hz, 3H). MALDI-TOFMS (AXIMA-TOF²) *m*/*z*[M+Na]⁺calcd. for C₉₃H₁₂₇NO₃₃S₃Na 1904.7, found1904.9.

### (Preparation of compound 90)

Compound 89 (403.4 mg, 214 µmol) was dissolved in 1,4-dioxane (18.4 mL) and purified water (3.05 mL), and 10% Pd/C (482.2 mg, 204 µmol) was added at room temperature. The reaction solution was stirred at room temperature under hydrogen atmosphere for 21.5 hours, and then diluted with a mixture of (chloroform:methanol = 1:1). The solid matter was filtered off with celite, and washed with a mixture of (chloroform:methanol = 1: 1). The filtrate and the washing solution were combined and concentrated under reduced pressure. The residue was purified by silica gel column chromatography (chloroform:methanol = 10:1) to obtain colorless solid compound 90 (265.9 mg, 77%). 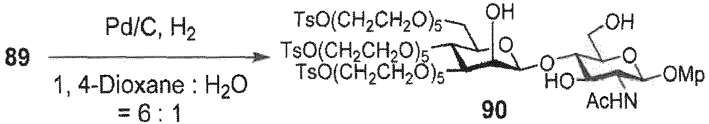 Compound 90 ¹H NMR (600 MHz,CDCl₃) δ:7.82-7.75 (m, 6H), 7.37-7.32 (m, 6H) , 6.94-6.90 (m, 2H), 6.80-6.76 (m, 2H) , 6.27 (d, J=8.2 Hz, 1H), 5.19 (d, J=8.2 Hz, 1H) , 4.80 (s, 1H), 4.58 (s, 1H), 4.18-4.10 (m, 7H) , 4.06 (t, J=9.3 Hz, 1H), 3.94 (dt, J=11.0, 4.5 Hz, 1H), 3.91-3.86 (m, 1H), 3.82-3.43 (m, 63H) , 3.39 (dd, J=8.2, 2.7 Hz, 1H) , 2.45 (s, 3H), 2.44 (s, 6H), 2.05 (brs, 3H), 1.99 (s, 3H) .
MALDI-TOFMS (AXIMA-TOF²) *m*/*z*[M+Na]⁺calcd. for C₇₂H₁₀₉NO₃₃S₃Na 1634.6, found1634.4.

### (Preparation of compound 91)

Compound 90 (265.9 mg, 165 µmol) was dissolved in dimethylformamide (3.30 mL), and sodium azide (171.4 mg, 2.64 mmol) was added at room temperature. The reaction solution was stirred at 50°C for 20 hours, diluted with toluene and methanol, and the solvent was removed under reduced pressure. The residue was azeotroped twice with toluene and dried in vacuo. The residue was purified by silica gel column chromatography (chloroform:methanol = 10:1) to obtain colorless solid compound 91 (176.6 mg, 87%). 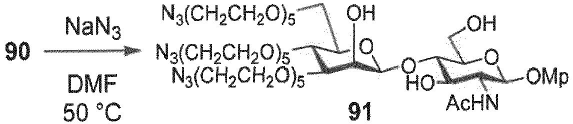 Compound 91 ¹H NMR (600 MHz, CDCl₃) δ:6.95-6.91 (m, 2H), 6.82-6.78 (m, 2H), 6.23 (d, J=7.6 Hz, 1H), 5.24 (d, J=8.9 Hz, 1H) , 4.79 (brs, 1H), 4.58 (s, 1H), 4.15-4.08 (m, 2H) , 3.94 (dt, J=11.0, 4.8 Hz, 1H), 3.91-3.87 (m, 1H), 3.83-3.55 (m, 60H), 3.50-3.46 (m, 2H), 3.41-3.34 (m, 7H) , 3.29 (s, 1H) , 2.29 (dd, J=8.6, 5.2 Hz, 1H), 2.01 (brs, 5H) . MALDI-TOFMS (AXIMA-TOF²) *m*/*z*[M+Na]⁺calcd. for C₅₁H₈₈N₁₀O₂₄Na 1247.6, found1247.3.

### (Preparation of compound 92)

Compound 91 (176.6 mg, 144 µmol) was dissolved in acetic anhydride (1.50 mL) and pyridine (1.50 mL). The reaction solution was stirred at room temperature for 18 hours, then diluted with chloroform, and the solvent was removed under reduced pressure. The residue was azeotroped with toluene three times and dried in vacuo. The residue was purified by silica gel column chromatography (chloroform:methanol = 20:1) to obtain colorless oily compound 92 (191.9 mg, 99%). 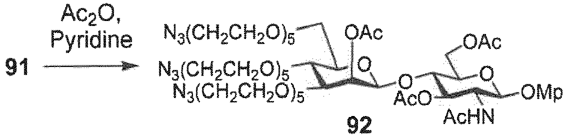 Compound 92 ¹H-NMR (600 MHz, CDCl₃) δ:6.95-6.89 (m, 2H), 6.82-6.76 (m, 2H), 5.84 (d, J=9.6 Hz, 1H), 5.40 (d, J=2.7 Hz, 1H), 5.21 (t, J=9.3 Hz, 1H) , 4.95 (d, J=7.6 Hz, 1H) , 4.48 (s, 1H), 4.36 (dd, J=12.4, 2.7 Hz, 1H), 4.28 (dd, J=12.0, 5.2 Hz, 1H) , 4.21 (dd, J=17.5, 9.3 Hz, 1H), 3.97 (dt, J=11.0, 4.5 Hz, 1H), 3.91 (t, J=8.9 Hz, 1H), 3.82-3.32 (m, 68H) , 2.13 (s, 3H), 2.11 (s, 3H), 2.08 (s, 3H), 1.97 (s, 3H).
MALDI-TOFMS (AXIMA-TOF²) *m*/*z*[M+Na]⁺calcd. for C₅₇H₉₄N₁₀O₂₇Na 1373.6, found1373.4.

### (Preparation of compound 93)

Compound 92 (1.2 mg, 0.89 µmol) was dissolved in acetonitrile (142 µL) and purified water (35.5 µL), and ammonium hexanitratocerate (IV) (2.3 mg, 4.20 µmol) was added under ice-cold conditions. The reaction solution was stirred at 0°C for 1.5 hours, diluted with chloroform, saturated sodium hydrogen carbonate aqueous solution was added, and then the mixture was extracted with chloroform three times. The organic phase was dried with anhydrous sodium sulfate, the dried matter was separated by filtration, and the solvent was removed under reduced pressure. The residue was purified by silica gel column chromatography (chloroform:methanol = 20:1) to obtain colorless oily compound 93 (0.6 mg, 54%). 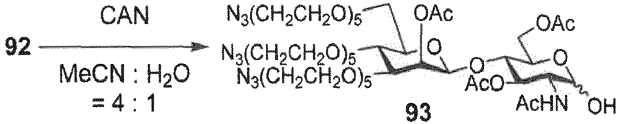 Compound 93 ¹H NMR (600 MHz, CDCl₃) δ:6.10 (s, 1H) , 5.90 (d, J=8.9 Hz, 1H) , 5.40-5.37 (m, 2H), 5.34 (d, J=2.7 Hz, 1H) , 5.17 (t, J=2.7 Hz, 1H), 4.55 (s, 1H), 4.37-4.14 (m, 8H), 4.00-3.84 (m,5H) ,3.83-3.34 (m,56H) ,2.11 (s,3H) ,2.10 (s,3H) ,2.09 (s,3H) ,1.96 (s,3H).
MALDI-TOFMS (AXIMA-TOF²) *m*/*z*[M+Na]⁺calcd. for C₅₀H₈₈N₁₀O₂₆Na 1267.6, found 1267.7.

### (Preparation of compound 94)

Compound 93 (0.6 mg, 0.482 µmol) was dissolved in dichloromethane (231 µL), and triethylamine (0.4 µL, 2.89 µmol) and 2-chloro-1,3-dimethylimidazolinium chloride (0.24 mg (0.24 mg, 1.45 µmol) dissolved in dichloromethane (10 µL) were added sequentially at room temperature. The reaction solution was stirred at room temperature for 1.5 hours, diluted with chloroform, saturated sodium hydrogen carbonate aqueous solution was added, and the mixture was extracted with chloroform three times. The organic phase was dried with anhydrous sodium sulfate, the dried matter was separated by filtration, and the solvent was removed under reduced pressure. The residue was purified by gel filtration chromatography (chloroform:methanol = 1:1, +1% triethylamine) to obtain crude product. The crude product was dissolved in methanol (241 µL), and 28% sodium methoxide methanol solution (5.0 µL) was added at room temperature. The reaction solution was stirred at room temperature for 24 hours, then diluted with methanol, and the preparation of compound 94 was confirmed by mass spectrometry. 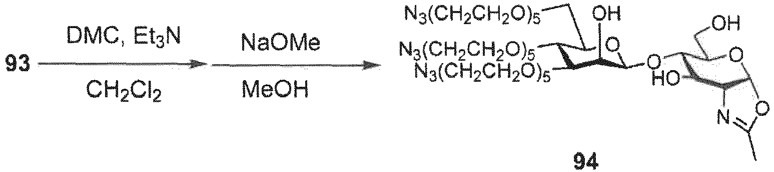 Compound 94 MALDI-TOFMS (AXIMA-TOF²) m/z[M+Na]⁺calcd. for C₄₄H₈₀N₁₀O₂₂Na 1123.5, found1122.9.

### «Example 12»

In this Example, rituximab to which a drug is conjugated was prepared.

### (Preparation of drug-conjugated rituximab)

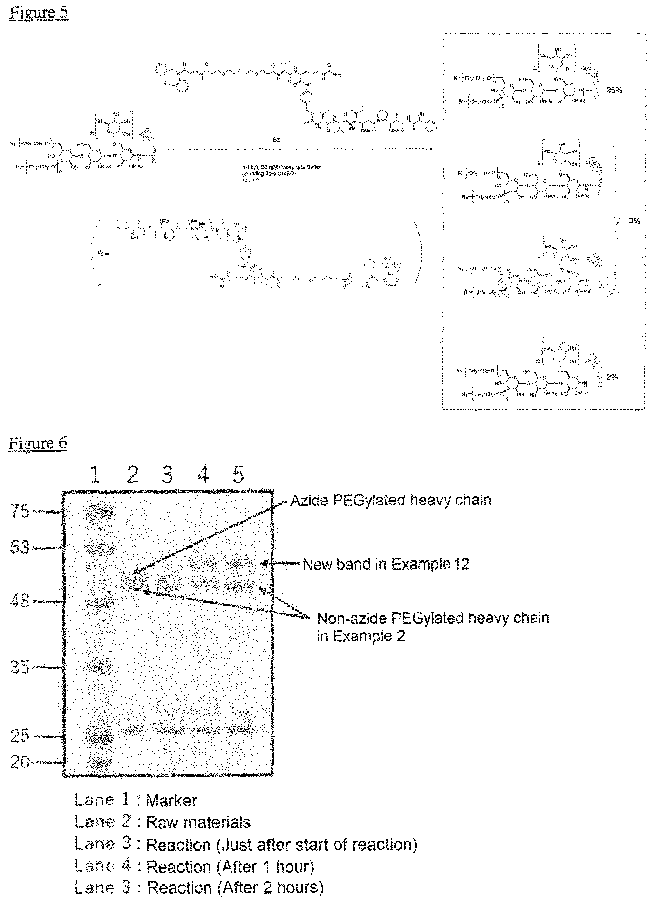

1.0 M pH 8.0 phosphate buffer solution (15.0 µL), water (165 µL), DMSO (76 µL), and 14.0 µL of DMSO solution (5 mM) of compound 52 (drug moiety is Monomethyl Auristatin E[MMAE]) were added to 30.0 µL of aqueous solution (7.0 µg/µL) of the reaction product obtained in Example 2. The reaction was carried out for 2 hours at room temperature in the dark. Mass spectrometry analysis showed that 95% of the products had the drug introduced into both azide groups of the heavy chain, 3% had the drug introduced into only one of the two positions, and 2% of the products were unreacted. A portion (3.3 µL) of the reaction solution was removed and examined by SDS-PAGE (Coomassie Brilliant Blue staining) using a 10% polyacrylamide gel, and it was confirmed that the band corresponding to the azido PEGylated heavy chain in the material was shifted to the high molecular weight side by the click reaction and that the drug was introduced into the reaction product obtained in Example 2 (Fig. 6).

### «Example 13»

In this Example, the cytotoxic effect of rituximab obtained in Example 12 was examined.

### (Examination of cell-killing effect of drug-conjugated rituximab)

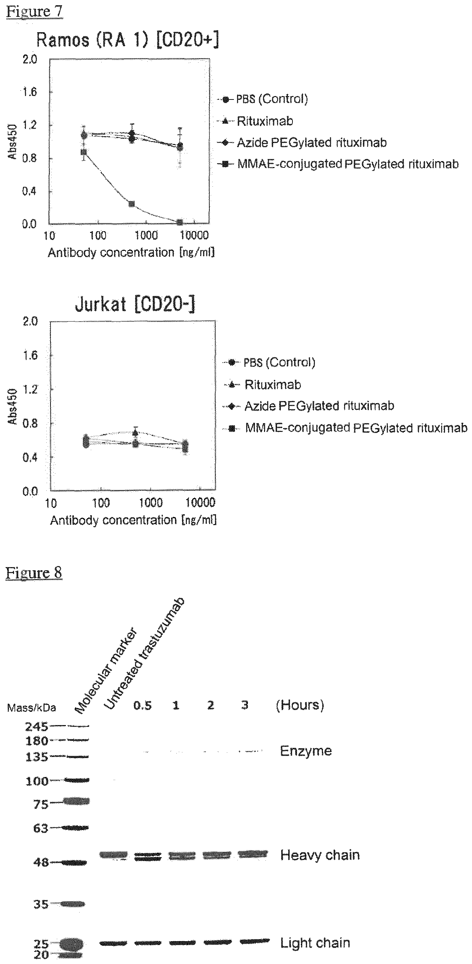

CD20-positive Ramos (RA1) cells and CD20-negative Jurkat cells were cultured in RPMI-1640 medium (Fujifilm Wako Pure Chemical Corporation) containing 10% SuperLowIgGFBS (Cytiva) and ZellShield (Minerva Biolabs Inc) as cultured cells. Ramos (RA1) cells or Jurkat cells suspended in culture medium were seeded into 96-well microplates (Coming inc) at 1.0×10⁴cells/90µL per well. A blank medium-only well was also prepared. The wells were incubated in the incubator at 37°C and 5% CO₂ overnight. The next day, 10 µL of control PBS, rituximab, azido PEGylated rituximab obtained in Example 2, or MMAE- conjugated rituximab obtained in Example 12 were added to each well, and the wells were incubated in the incubator at 37°C and 5% CO₂ for 72 hours. Antibody samples were diluted with PBS and added to the culture medium to a final concentration of 50, 500, or 5000ng/mL. After 72 hours of incubation, 10 µL of CellCountingKit-8 (Dojin Chemical Laboratory Co., Ltd.) was added, and the cells were incubated in the incubator at 37°C, and 5% CO₂ for another 2 hours. After 2 hours, absorbance at 450 nm was measured using a microplate reader SH-1300Lab (Corona Electric Co., Ltd.). Each concentration of each sample was measured for n=3, and the average absorbance was calculated after subtracting the absorbance of the blank. The results are shown in a semilogarithmic graph with the horizontal axis representing the sample concentration in a logarithmic scale and the vertical axis representing absorbance (Figure 7). For CD20-positive Ramos (RA1) cells, MMAE-conjugated rituximab reduced absorbance at antibody concentrations of 500 and 5000 ng/mL, indicating that the cells were killed. On the other hand, for CD20-negative Jurkat cells, the absorbance remained almost the same at each antibody concentration, and no clear cell-killing effect was observed.

### «Example 14»

In this Example, compound 12 was conjugated to trastuzumab.

### (Transfer reaction of compound 12 to the sugar chain binding site of trastuzumab (One-pot reaction. Another method of Example 2, which does not require prior preparation of antibody acceptor))

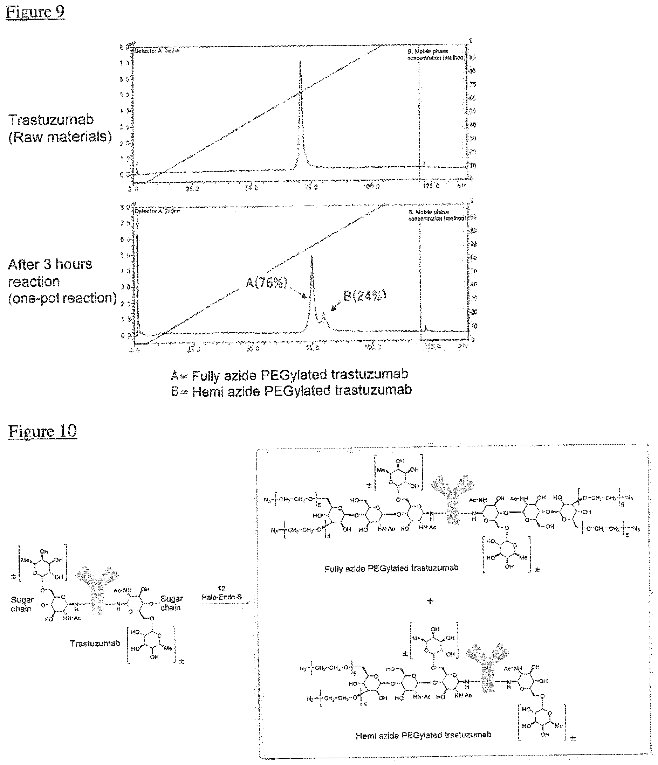

Trastuzumab (80 µg) manufactured by Chugai Pharmaceutical Co., Ltd., compound 12 (54 nmol) and the enzyme Halo-Endo-S wild type (8 µg) which was expressed and purified in E. coli as a host were added to 50 mM Tris-HCl buffer solution (pH 7.0). The total volume of 20 µL was kept at 37°C for 3 hours. At 0.5, 1, 2, and 3 hours, a portion of the reaction solution was removed and examined by SDS-PAGE (Coomassie Brilliant Blue staining) using a 10% polyacrylamide gel, and it was confirmed that a heavy chain band shifted to high molecular weight side by the transition of compound 12 in the reaction solution of 0.5 hour (Figure 8). A 76:24 mixture of Fully azido-PEGylated trastuzumab and Hemi-azido-PEGylated trastuzumab was obtained as the product of the transfer of compound 12 to the sugar chain binding site of trastuzumab in a 3-hour reaction (Figure 9). Mass spectrometry analysis showed that the transfer yield of compound 12 to the sugar chain moiety of the heavy chain was 87.4%.

### INDUSTRIAL APPLICABILITY

In the preparation of an antibody-drug conjugate, the sugar compound of the present invention prevents the degradation of antibodies and enables drugs to conjugate to antibodies efficiently.

## Claims

1. A sugar compound having a polyethylene glycol chain, of the following general formula (1). 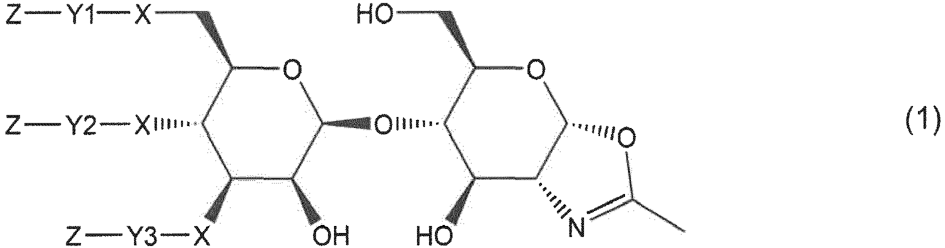 wherein each X is independently a single bond, an oxygen atom, -NH-, -COHN-, -COO-, or a group of formula (5) or (6), 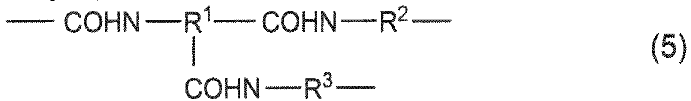 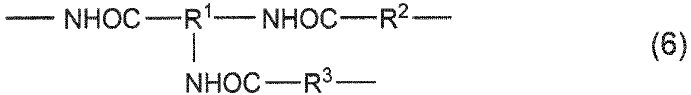 (wherein R¹ is trivalent branched hydrocarbon group having 1 to 6 carbon atoms, R² and R³ are each independently an alkylene group having 1 to 3 carbon atoms)
any one or more of Y1 to Y3 are present and are independently a PEG chain, a substituted PEG chain, or a PEG chain containing an oxygen atom, -NH-, -COHN-, or -COO- in the main chain, a structure of the PEG chain is linear or branched, and the number of branches is 2 to 10 in the case of branched structure,
Z is independently a hydroxy group, a methoxy group, an azido group, a tetrazine group which may be optionally substituted, a norbornene group, a trans-cyclooctene group, a dibenzocyclooctyne group, or a bicyclo[6.1.0]non-4-yn-9-ylmethyl group, or a cyanobenzothiazole group which may be optionally substituted, wherein at least one of Z is not a hydroxy group or methoxy group,
when any of Y1 to Y3 is not present, Z is hydrogen and X is oxygen,
when X is the group of formula (5) or (6), Y1 to Y3 are bonded to each of R² and R³ of the branched chains thereof,
when the PEG chain is branched structure, Z is bonded to each of branched chains.

2. The sugar compound having a polyethylene glycol chain according to claim 1, of the following general formula (2). 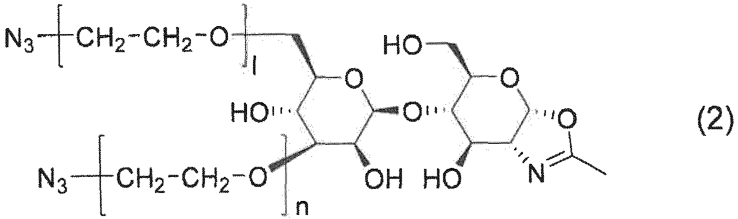 wherein 1 and n are each independently integers of 2 to 10.

3. The sugar compound having a polyethylene glycol chain according to claim 1, having a formula selected from the group consisting of the following formulas.

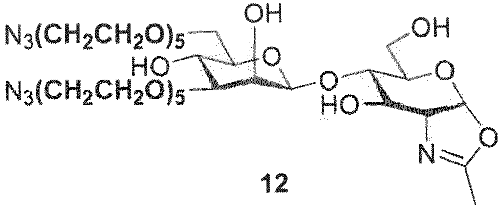

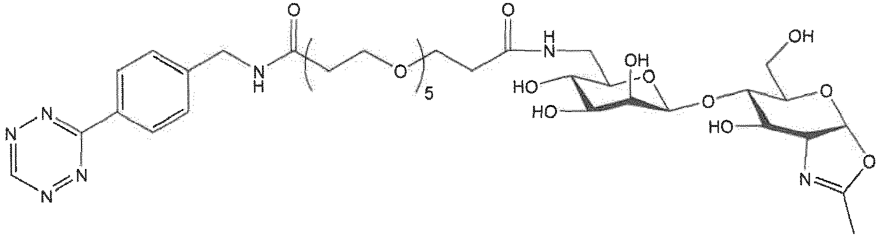

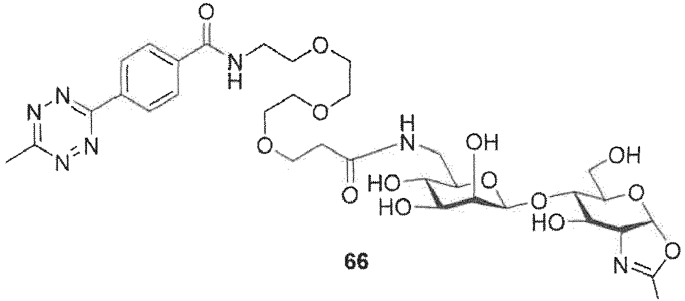

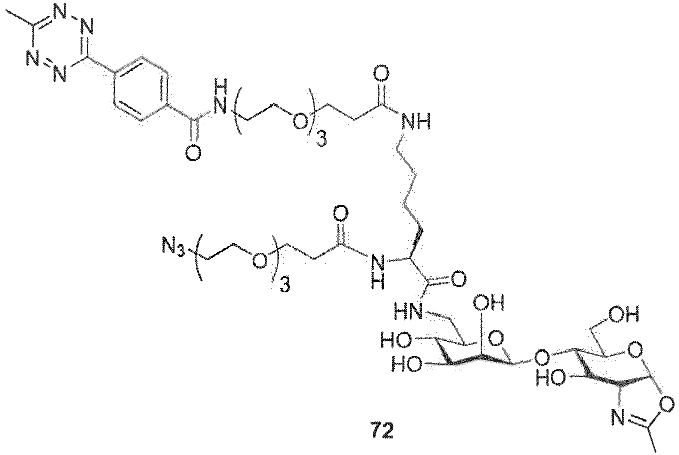

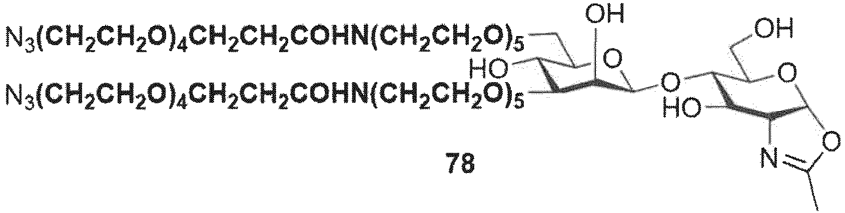

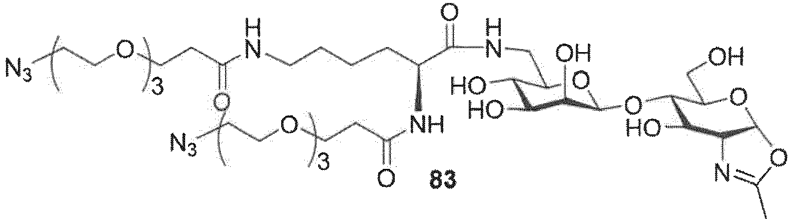

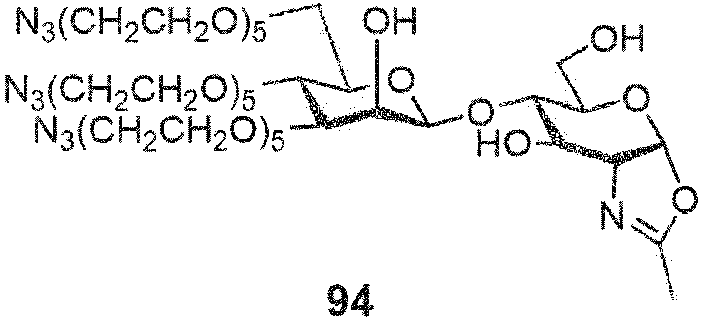

4. A modified immunoglobulin antibody with a sugar compound group having a polyethylene glycol chain represented by the following formula (7), which is derived from the sugar compound according to any one of claims 1 to 3, in a sugar-binding site of a side chain of an asparagine residue in a Fc region. 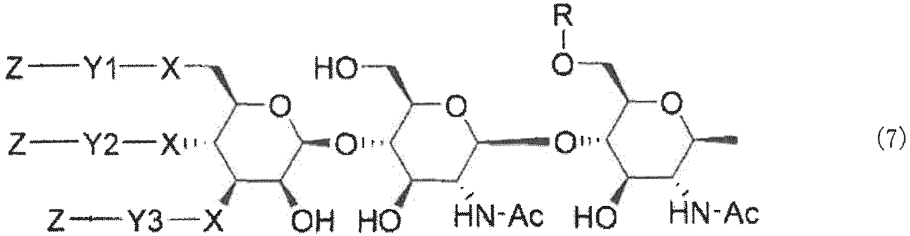 wherein X, Y1 to Y3, and Z are as above mentioned, and R is L-fucose or a hydrogen atom.

5. The modified immunoglobulin antibody according to claim 4, represented by the following formula (3), wherein the immunoglobulin antibody is an immunoglobulin G antibody, and the asparagine residue in the Fc region is the 297th asparagine residue in the Fc region. 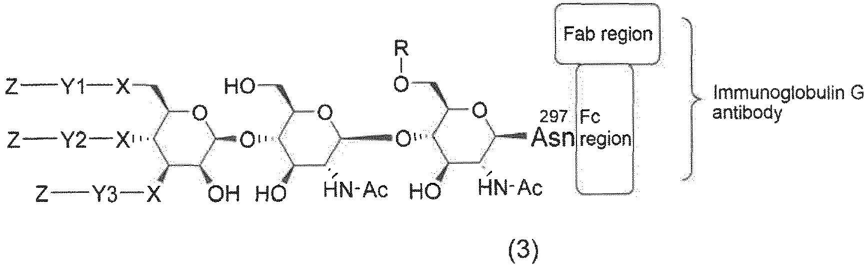 wherein X, Y1 to Y3, and Z are as above mentioned, and R is L-fucose or a hydrogen atom.

6. The modified immunoglobulin antibody according to claim 5, represented by the following formula (4), wherein the sugar compound having a polyethylene glycol chain is the sugar compound according to claim 2. 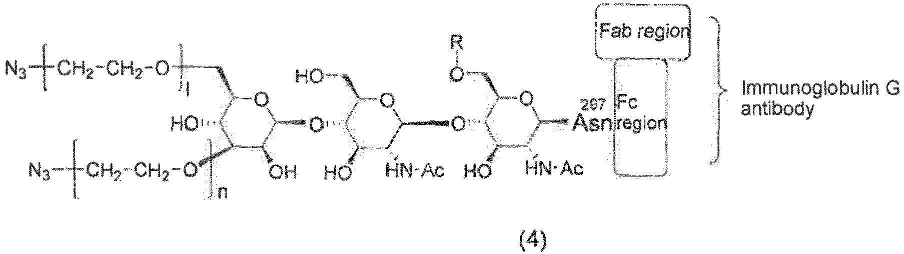 wherein R is L-fucose or a hydrogen atom, and 1 and n are integers of 2 to 10.
